(19) **Europäisches Patentamt** / **European Patent Office** / **Office européen des brevets**

(11) **EP 4 368 703 A1**

(12) **EUROPEAN PATENT APPLICATION**
published in accordance with Art. 153(4) EPC

(43) Date of publication:
**15.05.2024 Bulletin 2024/20**

(21) Application number: **22838040.8**

(22) Date of filing: **08.07.2022**

(51) International Patent Classification (IPC):
*C12N 5/0775* (2010.01)    *C12N 15/90* (2006.01)
*C12N 9/22* (2006.01)    *C12N 15/113* (2010.01)
*A61K 35/28* (2015.01)    *A61P 9/10* (2006.01)

(52) Cooperative Patent Classification (CPC):
**A61K 35/28; A61P 9/10; C12N 5/06; C12N 9/22;**
**C12N 15/113; C12N 15/90**

(86) International application number:
**PCT/KR2022/009918**

(87) International publication number:
**WO 2023/282688 (12.01.2023 Gazette 2023/02)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **09.07.2021 KR 20210090522**

(71) Applicant: **Toolgen Incorporated**
**Seoul 07789 (KR)**

(72) Inventors:
• **SHIN, Eun Ji**
**Seoul 07789 (KR)**

• **LEE, Kang In**
**Seoul 07789 (KR)**
• **CHOI, Yu Ri**
**Seoul 07789 (KR)**
• **SHIN, Hye Jung**
**Seoul 07789 (KR)**
• **LEE, Jae Young**
**Seoul 07789 (KR)**

(74) Representative: **Modiano, Gabriella Diana et al**
**Modiano & Partners (DE)**
**Steinsdorfstrasse, 14**
**80538 München (DE)**

Remarks:
The complete document including Reference
Table(s) and the Sequence Listing(s) can be
downloaded from the EPO website

(54) **MESENCHYMAL STEM CELL HAVING OXIDATIVE STRESS RESISTANCE, PREPARATION METHOD THEREFOR, AND USE THEREOF**

(57) Provided are mesenchymal stem cells, a preparation method therefor, and a use thereof, the mesenchymal stem cell having oxidative stress resistance by reducing or inhibiting the expression or activity level of KEAP1, which is a negative regulator of Nrf2, so as to increase the activity of Nrf2.

[Fig.7]

Target sequence (without PAM)

sgKeap1-BR-#5    ctgcatccaccacaacagtg
sgKeap1_exon3#23    ACAGCGACGGTTCTACGTCC

**Description**

[Technical field]

The present disclosure provides mesenchymal stem cells, a preparation method therefor, and a use thereof, the mesenchymal stem cells having oxidative stress resistance by reducing or inhibiting the expression or activity level of KEAP1, which is a negative regulator of Nrf2, so as to increase the activity of Nrf2.

[Background Art]

Mesenchymal stem cells have self-proliferation and multi-potency, and because there are various types of progenitor cells, it is easy to secure stem cell lines, and they are multipotent stem cells and has the advantage of being much more genetically stable than pluripotent stem cells such as embryonic stem cells. In addition, due to its anti-inflammatory and immunomodulatory properties, it has been developed as a cell therapeutic agent for cartilage regeneration, myocardial infarction treatment, and graft versus host disease treatment.

However, in lesion environments such as ischemic and inflammation in the body, due to poor blood supply, oxygen concentration is low (hypoxia) or oxidative stress caused by reactive oxygen species (ROS) increases, lowering the survival rate of mesenchymal stem cells in the body. In addition, in an oxidative stress environment, self-renewal of stem cells is inhibited, causing cellular senescence to accumulate, and aged mesenchymal stem cells show low therapeutic effectiveness.

In order to maintain and enhance the therapeutic effect of mesenchymal stem cells, which have a low survival rate in the body, repeated administration should be performed, but repeated administration can be a burden on the patient and a production rate must also be increased.

Reactive oxygen species (ROS) is a molecule naturally produced during cellular metabolism and plays an important role in the normal function of cells and signaling systems. However, excessive production of ROS induces oxidative stress and causes damage to intracellular molecules, interfering with the normal function and role of cells. Cells have a regulatory system that alleviates intracellular stress and toxicity caused by excessive reactive oxygen species (ROS), and a representative system is a nuclear factor (erythroid-derived 2)-like 2 (Nrf2)/Kelch-like ECH-associated protein 1 (Keap1) signaling system. Keap1, which plays a major role in regulating Nrf2, is present as a protein that regulates the antioxidant system in the human body, and protein-protein interaction (PPI) of Nrf2-Keap1 in the cytoplasm is an important factor in regulating antioxidant pathways that lead to inflammation and many pathological conditions caused by inflammatory mediators. Nrf2 is a transcription factor that protects cells from oxidative stress, and under normal conditions, it forms a complex with Keapl in the cytoplasm, but when Keap1 is inactivated by cytotoxic agents or oxidative stress, Nrf2 is released and enters the nucleus. Thereafter, Nrf2 is known to protect cells from oxidative damage by activating antioxidant enzymes such as HO-1, SOD, catalase, and GPx-1/2.

[Related Art Documents]

(Patent Document 1) Korean Patent Publication No. 10-2019-0069238

(Non-patent Document 1) Xiaozhen Dai et al., Trends Mol Med. 2020 Feb;26(2): 185-200, Nrf2: Redox and Metabolic Regulator of Stem Cell State and Function

(Non-patent Document 2) D S Yoon et al., Cell Death & Disease volume 7, pagee2093 (2016), Cellular localization of NRF2 determines the self-renewal and osteogenic differentiation potential of human MSCs via the P53-SIRT1 axis

(Non-patent Document 3) Yiling Hu et al., Front. Neurol., 18 February 2020, CRISPR/Cas9-Induced Loss of Keap1 Enhances Anti-oxidation in Rat Adipose-Derived Mesenchymal Stem Cells

(Non-patent Document 4) Mohammad Mohammadzadeh et al., Cell Stress and Chaperones volume 17, pages553-565 (2012), Nrf-2 overexpression in mesenchymal stem cells reduces oxidative stress-induced apoptosis and cytotoxicity

(Non-patent Document 5) Shouqin Zhang et al., J Cell Biochem. 2018 Feb;119(2):1627-1636, Nrf2 transfection enhances the efficacy of human amniotic mesenchymal stem cells to repair lung injury induced by lipopolysaccharide

[Disclosure]

[Technical Problem]

The present disclosure is for the purpose of providing mesenchymal stem cells with resistance to oxidative stress increased by reactive oxygen species (ROS) in lesion environments such as ischemic and inflammatory conditions in the body, a preparation method therefor, and a cell therapy using the same.

[Technical Solution]

In order to achieve the above matter, the present disclosure provides mesenchymal stem cells in which the expression or activity of KEAP1 protein, which binds to Nfr2 in the Nfr2-Keap1 pathway which is a regulatory system that alleviates intracellular stress and toxicity caused by excessive reactive oxygen species (ROS) and causes degradation, is reduced or suppressed.

The present disclosure also provides artificially engineered mesenchymal stems cells including an artificially engineered Kelch-like ECH-associated protein 1 (Keap1) gene, wherein the artificially engineered Keapl gene is different from KEAP1 gene sequence of a wild-type mesenchymal stem cell, the artificially engineered Keapl gene includes one or more indels in the nucleic acid sequence, the indel is located within a protospacer-adjacent motif (PAM) sequence in the exon 2 region or exon 3 region of KEAP1 gene, or within a contiguous sequence of 5 to 50 nucleotides adjacent to the 5' or 3' end of the PAM sequence, and the artificially engineered mesenchymal stems cells are characterized by improved oxidative stress resistance.

In addition, the present disclosure provides a composition for preparing mesenchymal stem cells with oxidative stress resistance, the composition including a guide nucleic acid including a guide sequence capable of targeting a target sequence of KEAP1 gene of mesenchymal stem cells, or a nucleic acid sequence encoding the same; and an editor protein or a nucleic acid sequence encoding the same.

The present disclosure also provides a preparation method of mesenchymal stem cells with oxidative stress resistance, including: (1) introducing a composition for preparing mesenchymal stem cells with the oxidative stress resistance into isolated mesenchymal stem cells; and (2) editing □□□□1 gene to reduce or suppress the expression or activity of □□□□1 protein by generating an indel in a target sequence of KEAP1 gene located in the genome of the mesenchymal stem cells.

In addition, the present disclosure provides a pharmaceutical composition for preventing or treating an ischemic disease, the composition comprising the artificially engineered mesenchymal stem cells to have the oxidative stress resistance as an active ingredient.

The present disclosure also provides a method of treating an ischemic disease, the method including administering to a mammal a therapeutically effective amount of the artificially engineered mesenchymal stem cells to have the above-described oxidative stress resistance.

The present disclosure also a use of mesenchymal stem cells artificially engineered to have the above-described oxidative stress resistance in the manufacture of a medicament for use in the prevention or treatment of an ischemic disease in a mammal.

[Advantageous Effects]

In the artificially engineered mesenchymal stem cells of the present disclosure, the gene encoding KEAP1 protein, which binds to Nrf2 and causes degradation, is artificially modified using genome editing technology, thereby reducing or suppressing the expression or activity of KEAP1 protein to increase the activity of Nrf2. Mesenchymal stem cells with reduced or suppressed expression or activity of KEAP1 protein exhibits resistance to oxidative stress increased by reactive oxygen species (ROS) in lesion environments such as ischemic and inflammation in the body, and can be used as a cell therapy with improved therapeutic effects on the ischemic disease by increasing survival rate and inhibiting aging in the body.

[Brief Description of Drawings]

FIG. 1 is a graph showing the results of measuring the indel efficiency for each guide RNA targeting the target sequence of SEQ ID NOs: 1 to 15 of Keapl gene using a targeted deep sequencing method.

FIG. 2 shows targeted deep sequencing of KEAP1 KO MSCs artificially engineered at position SEQ ID NO: 15 (sgKeap1-BR-#5) among the target sequences of KEAP1 gene, and shows the top sequence patterns in representative sequencing results.

FIG. 3 is a graph showing the indel frequency and expression level of KEAP1 mRNA in mesenchymal stem cells in which KEAP1 gene was knocked out using the screened Keapl target sgRNA, and a photo comparing the cell characteristics of mesenchymal stem cells according to gene editing.

FIG. 4 is a graph showing the results of measuring the indel efficiency of sgRNA for the target sequence of the NRF2 gene using a targeted deep sequencing method.

FIG. 5 is a graph showing the results of measuring the indel efficiency for each guide RNA targeting the target sequence of SEQ ID NOs: 49 to 56 of Keapl gene using a targeted deep sequencing method.

FIG. 6 is a graph showing comparison of the expression level of mRNA transcribed from KEAP1 gene in mesenchymal stem cells in which KEAP1 gene was knocked out using sgRNA targeting each target sequence of KEAP1 gene using qRT-PCR.

FIG. 7 shows the target sequences of SEQ ID NO: 15 and SEQ ID NO: 50 in KEAP1 gene.

FIG. 8 shows the indel efficiency of each target sequence for each guide RNA targeting the target sequences of SEQ ID NO: 15 and SEQ ID NO: 50 in KEAP1-knocked out mesenchymal stem cell □6 (FIG. 8(a)); and the expression level of mRN□ transcribed from KEAP1 gene measured using qRT-PCR in KEAP1-knocked out mesenchymal stem cells P6 and P7 (FIG. 8(b)).

FIG. 9 is a graph showing the survival rate of KEAP1 knockout mesenchymal stem cells in a normal environment (FIG. 9(a)) and an oxidative stress environment (FIG. 9(b)) using CCK8 analysis.

FIG. 10 is a graph showing the survival rate in an oxidative stress environment of KEAP1 knockout mesenchymal stem cells and mesenchymal stem cells in which NRF2 exon 2, a KEAP1 interacting domain, has been skip edited.

FIG. 11 is a graph showing the survival rate of KEAP1 knockout mesenchymal stem cells in an oxidative stress environment according to differences in target sequence.

FIG. 12 is a graph showing the survival rate of KEAP1 knockout mesenchymal stem cells P7 using SEQ ID NO: 15 and SEQ ID NO: 50 as target sequences in an oxidative stress environment.

FIG. 13 shows the survival rate (FIG. 13(a)), growth rate (FIG. 13(b)), population doubling level (PDL) (FIG. 13(c)), and population doubling time (PDT) (FIG. 13(d)) of stem cells knocking out the target sequences of SEQ ID NO: 15 (sgKeap1-BR#5) and SEQ ID NO: 50 (sgKEAP1-exon3-#23).

Figure 14 is a graph observing the degree of cell proliferation and cell cycle of KEAP1 knockout mesenchymal stem cells by BrdU analysis.

FIG. 15 is a graph showing the results of telomere length analysis of KEAP1 knockout mesenchymal stem cells.

FIG. 16 is a graph showing the results of confirming cytokines with increased expression in KEAP1 knockout mesenchymal stem cells through cytokine analysis.

FIG. 17 is a photograph and graph showing the results of collecting, staining, and analyzing lung tissue after IV administration of KEAP1 knockout MSCs to mice.

[Best Mode for Implementation of the Invention]

Hereinafter, the present disclosure will be described in more detail.

As used herein, the term "about" refers to quantity, level, value, number, frequency, percentage, dimension, size, amount, weight, or length that vary by 30, 25, 20, 25, 10, 9, 8, 7, 6, 5, 4, 3, 2 or 1 degrees with respect to a reference quantity, level, value, number, frequency, percentage, dimension, size, amount, weight, or length.

As used herein, the term "artificially engineered" is a term used to distinguish substances, molecules and the like that have a composition that is already present in the natural world, and means that artificial modifications have been made to the substances, molecules and the like. For example, the expression "artificially engineered gene" refers to a gene in which artificial modifications have been made to the composition of genes that are present in the natural world. In addition, the above term includes all meanings that may be recognized by a person skilled in the art, and may be appropriately interpreted depending on the context.

As used herein, the term "wild-type" refers to that a gene including a naturally occurring base sequence and a protein expressed from the gene have normal functional characteristics. Wild-type genes have a form in which no natural or artificial mutations have occurred and are most frequently observed in the population. When the term "wild-type" is used herein in contrast to artificially engineered genes and/or artificially engineered cells, this may be interpreted to mean a gene including an artificially engineered gene and/or a homologous "non-artificially engineered" naturally occurring base sequence corresponding to the artificially engineered cell, and a cell having the same. In addition, the above term includes all meanings that may be recognized by a person skilled in the art, and may be appropriately interpreted depending on the context.

As used herein, the expression "knock-out" or "knocked out gene" means that a mutation or artificial modification occurs in a wild-type gene, and as a result, means that the protein expressed by the wild-type gene is not produced through transcription and/or translation processes. For example, cells including knocked-out gene A may be unable to express the mRNA and/or protein expressed by wild-type gene A. Cells including a knocked-out gene A may be one in which only one gene A present in the cell is knocked out, or two or more genes A may be knocked out. In addition, the above term includes all meanings that may be recognized by a person skilled in the art, and may be appropriately interpreted depending on the context.

As used herein, the expression "knock-down" or "knocked down gene" means that a mutation or artificial modification occurs in a wild-type gene, resulting in the expression of a material in a lower amount than the wild-type gene. For example, cells including knocked down gene A may express less mRNA than that expressed by the wild-type gene A. As another example, cells including knocked down gene A may express a smaller amount of protein than the protein expressed by the wild-type gene A. Cells in which gene A has been knocked down may have only one gene A present in the cells knocked down, or have two or more genes knocked down. In addition, the above term includes all meanings that may be recognized by a person skilled in the art, and may be appropriately interpreted depending on the context.

As used herein, "expression reduction" means showing a lower level of expression than the expression level of mRNA and/or protein measured in the wild-type. The reduction may be a reduction of about 5% or more, about 10% or more, about 15% or more, about 20% or more, about 30% or more, about 50% or more, about 60% or more, about 70% or more, or about 100% or more compared to cells without genetic modification or wild-type cells.

As used herein, the term "activity reduction" or "reduced activity" may mean a reduction in relative activity when measuring protein or enzyme activity. Specifically, "activity reduction" or "reduced activity" means lower levels of protein, or enzyme activity compared to given parental or wild-type cells.

As used herein, the term "stem cell" refers to a broad concept that collectively refers to undifferentiated cells with sternness, that is, the ability to differentiate into various types of body tissue cells. At this time, the stem cells may be induced pluripotent stem cells, embryonic stem cells, and adult stem cells. In addition, the cells may be of human origin, but are not limited thereto.

As used herein, the expression "mesenchymal stem cell" is undifferentiated stem cells isolated from human or mammalian tissues, and may be derived from various tissues. In particular, the mesenchymal stem cells may be umbilical cord-derived mesenchymal stem cells, umbilical cord blood-derived mesenchymal stem cells, bone marrow-derived mesenchymal stem cells, fat-derived mesenchymal stem cells, muscle-derived mesenchymal stem cells, nerve-derived mesenchymal stem cells, skin-derived mesenchymal stem cells, amniotic membrane-derived mesenchymal stem cells, amniotic fluid-derived mesenchymal stem cells, perinatal tissue-derived mesenchymal stem cells, or placenta-derived mesenchymal stem cells, and technologies for isolating stem cells from each tissue are already known in the art.

The present disclosure provides mesenchymal stem cells in which the expression or activity of KEAP1 protein is reduced or suppressed.

In the mesenchymal stem cells with reduced or suppressed expression or activity of KEAP1, as the expression or activity of KEAP1 protein, which binds to Nfr2 in the Nfr2-Keap1 pathway which is a regulatory system that alleviates intracellular stress and toxicity caused by excessive reactive oxygen species (ROS) and causes degradation, is reduced or suppressed, the activity of Nrf2 increases, and exhibits resistance to oxidative stress increased by reactive oxygen species (ROS) in lesion environments such as ischemic and inflammation in the body. When mesenchymal stem cells with reduced or suppressed expression or activity of KEAP1 protein are used as a cell therapy, the survival rate of mesenchymal stem cells in the body increases and aging is inhibited, which can lead to improved therapeutic effects compared to wild-type mesenchymal stem cells or mesenchymal stem cells selected by related techniques.

Specifically, the present disclosure provides artificially engineered mesenchymal stem cells with oxidative stress resistance in which since KEAP1 gene in mesenchymal stem cells is artificially manipulated, the expression or activity of KEAP1 mRNA and/or KEAP1 protein is reduced or suppressed, and the cells are characterized by high viability in an oxidative stress environment.

The present disclosure provides artificially engineered mesenchymal stems cells including an artificially modified nucleic acid sequence of KEAP1 gene.

In the present disclosure, "artificial modification" or "artificial engineering" of a gene nucleic acid sequence may be achieved through modification of the nucleic acid sequence constituting the gene or chemical modification of a single base. This may be due to mutation, substitution, or deletion of part or all of the gene, or the insertion of one or more bases into the gene, and may be achieved using gene editing technology such as the CRISPR-enzyme system. As an example, artificial modification of the gene nucleic acid sequence may be achieved by non-homologous end joining (NHEJ) or homology directed repair (HDR) mechanisms.

As an example, the artificially engineered mesenchymal stems cells may have KEAP1 gene knocked out.

As used herein, "non-homologous end joining (NHEJ)" is a method of restoring or repairing a double-strand break in DNA by joining both ends of a cut double-strand or single strand together, and when two compatible ends, usually formed by a break in the double strand (for example, cleavage), repeat frequent contacts such that the two ends become fully joined, broken double strands are repaired.

In the process of repairing damaged genes or nucleic acids using NHEJ, some "insertion and/or deletion" (or "indels", InDels) of nucleic acid sequences may occur at the NHEJ repair site, and the gene in which an indel occurred does not have the same sequence as the wild-type gene. This insertion and/or deletion changes the reading frame of the gene, produce frameshifted transcript mRNA, and eventually loses its original function by undergoing nonsense-mediated decay or failing to synthesize normal proteins. In addition, mutations may occur that maintain the reading frame but insert or delete significant amounts of sequence, destroying the functionality of the protein. As another example, when an indel occurs in a transcriptional regulatory region such as the promoter region or enhancer region of a gene, the mRNA may not be transcribed or the transcription amount may be reduced, and thus the protein may not be expressed or the expression amount may be reduced. Alternatively, the mutagenesis mechanism of NHEJ may be utilized to delete only some sequence motifs when the generation of a specific final sequence is not required. For example, when two or more guide RNAs targeting the 5' and 3' intron regions of a specific exon are used to cause a double-strand break in each intron, and only part of the exon of the gene is deleted by NHEJ, other portions can be expressed normally and the main functionality of the protein may be maintained.

Using this NHEJ, the gene of interest may be specifically knocked out or knocked down using genome editing technology.

For example, CRISPR enzymes such as Cas9 or Cpf1, a type of genetic scissors, are used to cut double strands or two single strands of the target gene or target nucleic acid, indels are generated by NHEJ on a double strand or two single strands of a broken target gene or a broken target nucleic acid, and through this, specific knock-out or knock-down of the target gene or nucleic acid may be induced.

As an example, the artificially engineered mesenchymal stems cells may have KEAP1 gene knocked down.

In one embodiment of the present disclosure, KEAP1 gene of the artificially engineered mesenchymal stem cells may include one or more indels in the nucleic acid sequence.

In one embodiment of the present disclosure, the indel may be generated to be located within a protospacer-adjacent motif (PAM) sequence in the exon 2 region or exon 3 region of KEAP1 gene or within a contiguous sequence of 5 to 50 nucleotides adjacent to the 5' or 3' end of the PAM sequence.

In one embodiment of the present disclosure, the sequence of KEAP1 gene of the artificially engineered mesenchymal stem cells may not include one or more sequences selected from the group consisting of SEQ ID NOs: 1 to 15 and SEQ ID NOs: 49 to 56.

In one embodiment of the present disclosure, the artificially engineered mesenchymal stem cells may not express Keapl mRNA.

In one embodiment of the present disclosure, the mRNA transcribed from the artificially engineered Keapl gene of the artificially engineered mesenchymal stem cells may have lower expression levels of that mRNA compared to the level of mRNA transcribed from KEAP1 gene of wild-type mesenchymal stem cells.

In one embodiment of the present disclosure, the artificially engineered mesenchymal stem cells may have different Keapl mRNA sequences compared to wild-type stem cells.

In one embodiment of the present disclosure, the artificially engineered mesenchymal stem cells may have reduced expression or activity of Keapl protein compared to wild-type stem cells. Through this, the function of Keapl protein in the stem cells of the present disclosure may be reduced or lost.

That is, in the artificially engineered mesenchymal stem cells, the expression or activity of Keapl protein may be reduced by about 20% or more, about 30% or more, about 40% or more, about 50% or more, about 55% or more, about 60% or more, about 70% or more, about 75% or more, about 80% or more, about 85% or more, about 90% or more, about 95% or more, or about 100% compared to the expression or activity of wild-type mesenchymal stem cells.

The present disclosure provides a method of preparing artificially engineered mesenchymal stem cells with oxidative stress resistance, the method including reducing the expression or activity of Keapl protein of mesenchymal stem cells.

The increase or decrease in the expression or activity of Keapl protein may be achieved by artificial modification of KEAP1 gene, for example, using gene editing technology.

As an example, the genome editing technology may utilize TALEN (transcription activator like effctor nuclease) in which a transcription activator-like (TAL) effector (TALE) domain and a cutting domain are fused, a zinc-finger nuclease, or CRISPR-enzyme system derived from a clustered regularly interspaced short palindromic repeats (CRISPR), which is a microbial immune system, but is not limited thereto.

The entire contents disclosed in International Patent Publication No. WO2012/093833 or U.S. Patent Publication No. 2013-0217131 with respect to the above TALEN are incorporated in the present specification by reference. In relation to the above ZFN, Beerli et al. (2002) Nature Biotechnol. 20:135-141; □abo et al. (2001) □nn. Rev. Biochem. 70:313-340; Isalan et al, (2001) Nature Biotechnol. 19: 656-660; Segal et al. (2001) Curr. Opin. Biotechnol. 12:632-637; Choo et al. (2000) Curr. Opin. Struct. Biol.10:411-416; and U.S. Patent Nos. 7,888,121, 8,409,861, 6,479,626, 6,903,185, and 7,153,949 may be included as reference material in the present specification.

The "CRISPR-enzyme system" consists of a guide nucleic acid and/or an editor protein.

"Guide nucleic acid" refers to a nucleic acid capable of recognizing a target nucleic acid, target gene, or target chromosome and interacting with an editor protein. At this time, the guide nucleic acid may form a complementary bond with some nucleotides in the target nucleic acid, target gene, or target chromosome.

The guide nucleic acid may be in the form of a target DNA-specific guide RNA, DNA encoding the guide RNA, or a DNA/RNA mixture.

The guide nucleic acid may be a guide RNA. As an example, the "guide RNA" may be transcribed in vitro, particularly from an oligonucleotide double strand, or a plasmid template. As another example, the guide RNA may be encoded in the form of a vector, and may be transferred into cells in an ex vivo or in vivo environment and transcribed from the vector, but is not limited thereto.

The design and composition of the guide RNA are known to those skilled in the art and are described in detail in Korean Registration Patents 10-1656236, 10-1656237, 10-1706085, 10-2052286, and 10-2182847, and the entirety of the above Registration Patents is incorporated herein as reference material for the present disclosure.

The guide nucleic acid may include a scaffold sequence portion and a guide sequence portion. The scaffold sequence portion is a portion that interacts with the Cas protein and allows the Cas protein and a guide nucleic acid to bind to form a complex (ribonucleoprotein, RNP). Generally, the scaffold sequence portion includes tracrRNA and some sequence portions of crRNA, and the scaffold sequence is determined depending on which Cas protein is used.

The guide sequence portion is a nucleotide sequence portion that may bind complementary to some sequences of any one of the double strands of a target gene or nucleic acid, and a nucleotide sequence portion that may be artificially modified, and is determined by the target nucleotide sequence of interest. At this time, the guide sequence may be a nucleotide sequence having at least 50%, 55%, 60%, 65%, 70%, 75%, 80%, 85%, 90%, or 95% or more complementarity or complete complementarity with the guide nucleic acid binding sequence of the target gene or target nucleic acid. The guide sequence may be a sequence included in the guide domain of the guide nucleic acid.

The guide sequence portion may be included in crRNA. As an example, the guide nucleic acid may be a dual RNA including two RNAs, namely, crRNA (CRISPR RNA) and tracrRNA (trans-activating crRNA) as components.

As another example, the guide nucleic acid may be a single-chain guide RNA (sgRNA) in which the main portions of crRNA and tracrRNA are linked.

The target sequence may be a nucleotide sequence of a certain length present in the target gene or target nucleic acid, and specifically, some nucleotide sequences within the target region classified by a regulatory region of the target gene, coding region (or CDS, coding sequence), or a non-coding region (or untranslated region (UTR)), or one or more some nucleotide sequences selected from a combination of the target regions. The target sequence may be a target of a guide nucleic acid-editor protein complex (RNP).

In one embodiment of the present disclosure, the target sequence may be a sequence included in a second region or third region of the exon of the wild-type KEAP1 gene.

In one embodiment of the present disclosure, the target sequence is one or more sequences selected from SEQ ID NOs: 1 to 15.

The target sequence is a nucleotide sequence adjacent to a protospacer-adjacent motif (PAM) sequence recognized by the editor protein, and may include all or part of the PAM, but is not limited thereto.

The term "target sequence" may be used to mean both types of nucleotide sequence information. For example, in the case of a target gene, the target sequence may mean sequence information of the transcribed strand of the target gene DNA, or may mean nucleotide sequence information of the non-transcribed strand.

The target sequence includes a guide nucleic acid binding sequence or a guide nucleic acid non-binding sequence. "Guide nucleic acid binding sequence" is a nucleotide sequence that has partial or complete complementarity with the guide sequence included in the guide domain of a guide nucleic acid, and may bind complementary to the guide sequence included in the guide domain of the guide nucleic acid. The target sequence and guide nucleic acid binding sequence are nucleotide sequences that may vary depending on the target gene or nucleic acid, that is, depending on the subject to be genetically engineered or corrected, and the guide nucleic acid may be designed in various ways depending on the target gene or target nucleic acid.

The guide nucleic acid non-binding sequence is a nucleotide sequence that has partial or complete complementarity with the guide sequence included in the guide domain of the guide nucleic acid, and cannot bind complementary to the guide sequence included in the guide domain of the guide nucleic acid. In addition, the guide nucleic acid non-binding sequence is a nucleotide sequence that is complementary to the guide nucleic acid binding sequence and may bind complementary to the guide nucleic acid binding sequence. The guide nucleic acid binding sequence is some nucleotide sequences of the target sequence, and may be a nucleotide sequence having two different sequence orders of the target sequence, that is, one nucleotide sequence of two nucleotide sequences capable of complementary binding to each other. At this time, the guide nucleic acid non-binding sequence may be the remaining nucleotide sequence of the target sequence excluding the guide nucleic acid binding sequence.

The guide nucleic acid binding sequence may be a target sequence, that is, one nucleotide sequence selected from a nucleotide sequence identical to the transcribed strand and a nucleotide sequence identical to the non-transcribed strand. At this time, the guide nucleic acid non-binding sequence may be the guide nucleic acid binding sequence among the target sequences, that is, the remaining nucleotide sequence excluding one nucleotide sequence selected from the nucleotide sequence identical to the transcribed strand and the nucleotide sequence identical to the non-transcribed strand.

The guide nucleic acid binding sequence may be the same length as the target sequence. The guidenucleic acid non-binding sequence may be the same length as the target sequence or guidenucleic acid binding sequence. The guide nucleic acid binding sequence may be 5 to 50 nucleotide sequences.

In one embodiment, the guide nucleic acid binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences. The guide nucleic acid non-binding sequence may be 5 to 50 nucleotide sequences.

In one embodiment, the guide nucleic acid non-binding sequence is 16 nucleotide sequences, 17 nucleotide sequences, 18 nucleotide sequences, 19 nucleotide sequences, 20 nucleotide sequences, 21 nucleotide sequences, 22 nucleotide sequences, 23 nucleotide sequences, 24 nucleotide sequences, or 25 nucleotide sequences.

The guide nucleic acid binding sequence may form a partial or complete complementary bond with the guide sequence included in the guide domain of the guide nucleic acid, and the length of the guide nucleic acid binding sequence may be the same as the length of the guide sequence.

The guide nucleic acid binding sequence may be a nucleotide sequence complementary to the guide sequence included in the guide domain of the guide nucleic acid, for example, a nucleotide sequence that is at least 70%, 75%, 80%, 85%, 90%, or 95% complementary or completely complementary.

As an example, the guide nucleic acid binding sequence may have or include a 1 to 8 nucleotide sequences that is not complementary to the guide sequence included in the guide domain of the guide nucleic acid.

The guide nucleic acid non-binding sequence may have partial or complete homology to the guide sequence included in the guide domain of the guide nucleic acid, and the length of the guide nucleic acid non-binding sequence may be the same as the length of the guide sequence. As an example, the guide sequence may be designed based on a sequence having homology to the guide nucleic acid non-binding sequence.

The guide nucleic acid non-binding sequence may be a nucleotide sequence having homology to the guide sequence included in the guide domain of the guide nucleic acid, for example, a nucleotide sequence having at least 70%, 75%, 80%, 85%, 90%, or 95% homology.

As an example, the guide nucleic acid non-binding sequence may have or include a 1 to 8 nucleotide sequences that is not homologous to the guide sequence included in the guide domain of the guide nucleic acid. The guide nucleic acid non-binding sequence may bind complementary to the guide nucleic acid binding sequence, and the guide nucleic acid non-binding sequence may be the same as the length of the guide nucleic acid binding sequence.

The guide nucleic acid non-binding sequence may be a nucleotide sequence complementary to the guide nucleic acid binding sequence, for example, a nucleotide sequence that is at least 90% or 95% complementary or completely complementary.

As an example, the guide nucleic acid non-binding sequence may have or include 1 to 2 nucleotide sequences that are not complementary to the guide nucleic acid binding sequence. In addition, the guide nucleic acid binding sequence may be a nucleotide sequence located close to a complementary sequence complementary to a nucleotide sequence (PAM sequence) that may be recognized by the editor protein.

As an example, the guide nucleic acid binding sequence may be a contiguous 5 to 50 nucleotide sequences located adjacent to the 5' end or/and 3' end of a sequence complementary to a nucleotide sequence (PAM sequence) that may be recognized by the editor protein.

In addition, the guide nucleic acid non-binding sequence may be a nucleotide sequence located close to a nucleotide sequence (PAM sequence) that may be recognized by the editor protein.

As an example, the guide nucleic acid non-binding sequence may be a contiguous 5 to 50 nucleotide sequences located adjacent to the 5' end or/and 3' end of a nucleotide sequence (PAM sequence) that may be recognized by the editor protein.

"Editor protein" means a peptide, polypeptide or protein that either bind directly to nucleic acids, or do not bind directly, but may interact a nucleic acid. The editor protein is conceptually referred to as "artificially engineered nuclease" or RNA-guided endonuclease RGEN).

In one embodiment, the editor protein may be a CRISPR enzyme. "CRISPR enzyme" is the main protein component of the CRISPR-enzyme system, also called "Cas protein (CRISPR associated protein)", and refers to a nuclease that may recognize a target sequence and cleave DNA by mixing or forming a complex with a guide RNA.

CRISPR enzymes are known to those skilled in the art, see Korean Registration Patents Nos. 10-1656236, 10-1656237, 10-1706085, 10-2052286, and 10-2182847. The CRISPR enzyme is used herein as a concept that includes all variants that may act as an endonuclease or nickase activated in cooperation with a guide RNA, in addition to the native protein. In the case of activated endonuclease or nickase, target DNA may be cut and this may be used to perform genome editing. In addition, in the case of an inactivated variant, the genome editing may be used to regulate transcription or isolate the desired DNA.

The CRISPR enzyme is a nucleic acid or polypeptide (or protein) having a sequence encoding the CRISPR enzyme, typically, Type II CRISPR enzyme or Type V CRISPR enzyme is widely used, and the Type II CRISPR enzyme is CRISPR associated protein 9 (Cas9) protein.

The Cas9 protein may be derived from various microorganisms such as *Streptococcus pyogenes, Streptococcus thermophilus, Streptococcus* sp., *Campylobacter jejuni, Staphylococcus aureus, Staphylococcus Auricularis,* and *Neisseria meningitidis.*

In order for the Cas9 protein to induce a double-stranded DNA break, the Cas9 protein recognizes a protospacer-adjacent motif (PAM) sequence, which is a nucleotide sequence of a certain length, and requires that a portion of the guide RNA (the guide sequence portion) bind complementary to the complementary strand (the guide nucleic acid binding sequence) of a single strand of DNA (the guide nucleic acid non-binding sequence) on which the target sequence is located.

This PAM sequence is a sequence determined depending on the type or origin of the Cas9 protein, for example, a *Streptococcus pyogenes*-derived Cas9 protein (SpCas9) may recognize the 5'-NGG-3' sequence (complementary sequence: 5'-CCN-3') in the target nucleic acid. At this time, N is one of adenosine (A), thymidine (T), cytidine (C), and guanosine (G). In addition, SpCas9 can recognize the 5'-NAG-3' sequence (complementary sequence: 5'-CTN-3') in the target nucleic acid with low activity.

In addition, the Type V CRISPR enzyme includes Cpf1, and Cpf1 may be *Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter; Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opituaceae, uberibacillus, Bacillus, Brevibacilus, Methylobacterium,* or *Acidaminococcus*-derived Cpf1.

CRISPR enzymes such as the Cas9 or Cpf1 protein may be isolated from microorganisms present in nature or produced unnaturally through recombinant or synthetic methods. The Cas protein may also be in a form that is easy to introduce into cells. For example, the Cas protein may be linked to a cell-penetrating peptide or protein transduction domain. The protein transduction domain may be poly-arginine or HIV-derived TAT protein, but is not limited thereto. Since various types of cell-penetrating peptides or protein transduction domains are known in the art in addition to the examples described above, those skilled in the art are not limited to the above examples and may apply various examples to the present specification. In addition, the Cas protein may be fused with a functional domain such as a nuclear localization sequence or signal (NLS). In addition, the Cas9 protein may be encoded in the form of a vector and expressed within cells.

The present disclosure provides a composition for preparing mesenchymal stem cells with oxidative stress resistance, the composition including a guide nucleic acid including a guide sequence capable of targeting a target sequence of KEAP1 gene of mesenchymal stem cells, or a nucleic acid sequence encoding the same; and an editor protein or a nucleic acid sequence encoding the same.

In addition, the composition may optionally further include a donor including a specific nucleotide sequence to be inserted or a nucleic acid sequence encoding the same.

The donor refers to exogenous nucleotide sequences that may express a specific peptide or protein, and may be inserted into genomic DNA through homology directed repair (HDR).

The donor may be a double-stranded nucleic acid or a single-stranded nucleic acid. The donor may be linear or circular.

The donor may be in the form of a viral vector or a non-viral vector (for example, a plasmid).

The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus.

The viral vector may be one or more viral vectors selected from the group consisting of retrovirus, lentivirus, adenovirus, adeno-associated virus (AAV), vaccinia virus, poxvirus, and herpes simplex virus (HSV).

The target sequence may be a target of a guide nucleic acid-editor protein complex, and the target sequence may include a protospacer-adjacent motif (PAM) sequence recognized by the editor protein, but is not limited thereto.

The guide nucleic acid composition may include a guide domain capable of targeting the target sequence of KEAP1 gene.

In one embodiment of the present disclosure, the target sequence of the composition may be one or more sequences selected from SEQ ID NOs: 1 to 15.

In the present specification, the guide nucleic acid, editor protein or guide nucleic acid-editor protein complex (ribonucleoprotein, RNP) and/or donor may be delivered or introduced into the subject in various forms.

In this case, the "subject" refers to an organism into which the guide nucleic acid, editor protein, or guide nucleic acid-editor protein complex is introduced; an organism in which a guide nucleic acid, an editor protein, or a guide nucleic acid-editor protein complex operates; or a specimen or sample obtained from the organism.

The subject may be an organism including a target gene, a target nucleic acid, or a target chromosome of the guide nucleic acid-editor protein complex.

The organism may be an animal, animal tissue, or animal cell. At this time, the tissue may be the eye, skin, liver, kidney, heart, lung, brain, muscle, or blood.

The cells may be stem cells, liver cells, cardiac muscle cells, endothelial cells, or pancreatic cells.

The specimen or sample may be acquired from an organism including target gene, target nucleic acid, or target chromosome, such as saliva, blood, liver tissues, brain tissues, liver cells, nerve cells, phagocytes, macrophages, T cells, B cells, astrocytes, cancer cells, or stem cells.

The guide nucleic acid, editor protein, or guide nucleic acid-editor protein complex may be delivered or introduced into the subject in the form of DNA, RNA, or a mixture thereof.

At this time, DNA, RNA, or a mixture thereof encoding the guide nucleic acid and/or the editor protein may be delivered or introduced into the subject by methods known in the art.

Alternatively, the form of DNA, RNA, or a mixture thereof encoding the guide nucleic acid and/or the editor protein may be delivered or introduced into the subject by vector, non-vector, or a combination thereof.

The vector may be a viral vector or a non-viral vector (for example, a plasmid).

The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus.

The viral vector may be one or more selected from the group consisting of retrovirus, lentivirus, adenovirus, adeno-associated virus (AAV), vaccinia virus, poxvirus, and herpes simplex virus.

The non-vector may be naked DNA, DNA complex, or mRNA.

In one embodiment of the present disclosure, the nucleic acid sequence encoding the guide nucleic acid and/or the editor protein may be delivered or introduced into the subject in the form of one or more vectors.

The vector may include a guide nucleic acid and/or a nucleic acid sequence encoding an editor protein. As an example, the vector may simultaneously include a guide nucleic acid and a nucleic acid sequence encoding an editor protein. As another example, the vector may include a nucleic acid sequence encoding a guide nucleic acid. For example, the nucleic acid sequence encoding the guide nucleic acid may be all included in one vector, or the nucleic acid sequence encoding the guide nucleic acid may be divided and included in multiple vectors. As another example, the vector may include a nucleic acid sequence encoding an editor protein. For example, in the case of the editor protein, the nucleic acid sequence encoding the editor protein may be included in one vector, or the nucleic acid sequence encoding the editor protein may be divided and included in multiple vectors.

The editor protein may be delivered or introduced into the subject in the form of peptide, polypeptide, or protein.

The editor protein may be delivered or introduced into the subject in the form of peptide, polypeptide, or protein by methods known in the art.

The guide nucleic acid and the editor protein may be delivered or introduced into the subject in the form of a nucleic acid-protein mixture.

The guide nucleic acid and the editor protein may be delivered or introduced into the subject in the form of a guide nucleic acid-editor protein complex. For example, the guide nucleic acid may be DNA, RNA, or a mixture thereof. The editor protein may be in the form of peptide, polypeptide, or protein. As an example, in the case of the guide nucleic acid and the editor protein, a guide nucleic acid in the form of RNA and an editor protein in the form of a protein may be delivered or introduced into the subject in the form of a guide nucleic acid-editor protein complex, that is, ribonucleoprotein (RNP).

In addition, the present disclosure provides a preparation method of mesenchymal stem cells with oxidative stress resistance, the method including (1) introducing a composition for preparing mesenchymal stem cells with the oxidative stress resistance into isolated mesenchymal stem cells; and (2) editing □□□□1 gene to reduce or suppress the expression or activity of KEAP1 protein by generating an indel in a target sequence of KEAP1 gene located in the genome of the mesenchymal stem cells.

At this time, the "introduction" may be performed by one or more means selected from electroporation, lipofection, microinjection, genetic blueprint, liposomes, positive liposomes, plasmids, viral vectors, nanoparticles, protein translocation domain (PTD) Methods, immunoliposomes, polycations or lipids: nucleic acid conjugates, naked DNA, artificial virions, and preparation-enhanced uptake methods of DNA, but is not limited thereto.

In one embodiment of the present disclosure, mesenchymal stem cells with oxidative stress resistance may be prepared by introducing a composition for preparing mesenchymal stem cells with oxidative stress resistance into isolated mesenchymal stem cells by electroporation.

In one embodiment of the present disclosure, indels can be generated by contacting KEAP1 gene located within the genome of the mesenchymal stem cell with a CRISPR/Cas9 complex including Streptococcus pyogenes-derived Cas9 protein and guide RNA that may target the KEAP1 gene.

In one embodiment of the present disclosure, the target sequence may be one or more sequences selected from the group consisting of SEQ ID NOs: 1 to 15.

The artificially engineered mesenchymal stem cells with oxidative stress resistance of the present disclosure may be used as a cell therapy agent by being included as an active ingredient in a pharmaceutical composition for the prevention or treatment of ischemic diseases ischemic heart diseases, peripheral artery disease, critical limb ischemia (CLI), thromboangitis obliteran, diabetic peripheral angiopathy, osteonecrosis, mesenteric ischemia, ischemic colitis, ischemic enteritis, acute kidney injury, ischemia-reperfusion injury, ischemic hepatitis, ischemic pancreatitis, ischemic optic neuropathy, chronic obstructive pulmonary disease, acute respiratory distress syndrome (ARDS), COVID-19 infection, neonatal hypoxic-ischemic encephalopathy, or stroke.

The ischemia-reperfusion injury generally occurs when the blood supply to an organ or tissue is interrupted, resulting in reduced oxygen supply to the tissue, and when blood circulation is restored by reperfusion under ischemic conditions, and is a complex result of the cascade of reperfusion injury leading to an acute inflammatory response.

In the present specification, "cell therapeutic agent" is a medicine (US FDA regulations) used for the purposes of treatment, diagnosis, and prevention with cells and tissues prepared through isolation, culture, and special engineering from an individual, and refers to a medicine used for treatment, diagnosis, and prevention purposes through a series of actions such as changing the biological characteristics of cells by ex vivo proliferation selection or other methods of living autologous, allogeneic, or xenogeneic cells to restore cell or tissue function.

The route of administration of a cell therapeutic composition of the present disclosure may be administered via any general route as long as the desired tissue may be reached. The route may be administered parenterally, for example, intraperitoneally, intravenously, intramuscularly, subcutaneously, or intradermally, but is not limited thereto.

The composition may be formulated in a suitable form with a pharmaceutical carrier commonly used in cell therapy. The term "pharmaceutically acceptable" refers to a composition that is physiologically acceptable and does not usually cause allergic responses such as gastrointestinal disorders, dizziness, or similar responses when administered to humans. Pharmaceutically acceptable carriers include, for example, water, suitable oils, saline solutions, carriers for parenteral administration such as aqueous glucose and glycol, and may further include stabilizers and preservatives. The suitable stabilizer include antioxidants such as sodium bisulfite, sodium sulfite, or ascorbic acid. Suitable preservative include benzalkonium chloride, methyl- or propyl-paraben, and chlorobutanol. As for other pharmaceutically acceptable carriers, those described in the following literature may be referred to (Remington's Pharmaceutical Sciences, 19th ed., Mack Publishing Company, Easton, PA, 1995).

Additionally, the composition may be administered by any device that allows the cell therapeutic agent to move to target cells.

The cell therapeutic composition of the present disclosure may include a therapeutically effective amount of a cell therapeutic agent for the treatment of disease. The term "therapeutically effective amount" refers to an amount of an active ingredient or pharmaceutical composition that induces a biological or medical response in a tissue system, animal, or human, as believed by a researcher, veterinarian, physician or other clinician, and includes an amount that lead to alleviation of the symptoms of the disease or disorder being treated.

It is obvious to those skilled in the art that the cell therapeutic agent included in the composition of the present disclosure will vary depending on a desired effect. Therefore, an optimal cell therapeutic agent content may be easily determined by those skilled in the art, and may be adjusted depending on a variety of factors, including the type of disease, the severity of the disease, the content of other ingredients contained in the composition, the type of formulation, and the patient's age, weight, general health, gender and diet, administration time, administration route and secretion rate of the composition, treatment period, and concurrently used medications. Considering all of the above factors, it is important to include a minimum amount of the maximum effect without side effects. For example, the daily dosage of the stem cells of the present disclosure is $1.0 \times 10^5$ to $1.0 \times 10^{30}$ cells/kg of body weight, preferably $1.0 \times 10^{10}$ to $1.0 \times 10^{20}$ cells/kg of body weight, and may be administered once or in several divided doses. However, it should be understood that the actual dosage of the active ingredient should be determined in light of various related factors such as the disease to be treated, the severity of the disease, the route of administration, the patient's weight, age, and gender, and therefore, the dosage does not limit the scope of the present disclosure in any way.

In addition, the composition including the cell therapy agent of the present disclosure as an active ingredient may be administered through routes in the related art such as rectal, intravenous therapy (i.v.), intraarterial, intraperitoneal, intramuscular, intrasternal, transdermal, topical, intraocular or intradermal route.

The present disclosure provides a method of treating an ischemic disease, including administering to a mammal a therapeutically effective amount of the artificially engineered mesenchymal stem cells. As used herein, the term "mammal" refers to a mammal that is the subject of treatment, observation or experiment, preferably a human. At this time, an ischemic disease follows the above.

The present disclosure also use of the artificially engineered mesenchymal stem cells described above in the manufacture of a medicament for use in the prevention or treatment of an ischemic disease in a mammal. At this time, an ischemic disease follows the above.

Hereinafter, the present disclosure will be described in more detail through examples.

These examples are only for illustrating the present disclosure in more detail, and it will be apparent to those skilled in the art that the scope of the present disclosure is not limited by these examples.

**Example 1: Preparation of KEAP1 knocked-out mesenchymal stem cells**

sgRNA design

To knock out KEAP1 gene, among the guide sequences predicted on http://www.rgenome.net/cas-designer/, the target sequence of guide RNA with mismatch 1,0,0, which is expected to have less off-target effect, was selected.

The target sequence of KEAP1 gene is summarized in Table 1 below.

[Table 1]

| Classification | Exon | RGEN Target (5' to 3' direction) with PAM(underlined) | Indel (%) |
|---|---|---|---|
| sgKeap1-#1 | 2 | TGCAGTCACAGTGCCCTGAGGGG (SEQ ID NO: 1) | 18 |
| sgKeap1-#2 | 2 | GAAGGTGCGGTTGCCATGCTGGG (SEQ ID NO: 2) | 17.5 |
| sgKeap1-#3 | 2 | TGAAGGTGCGGTTGCCATGCTGG (SEQ ID NO: 3) | 7.9 |
| sgKeap1-#4 | 2 | GATCATACCAAGCAGGCCTTTGG (SEQ ID NO: 4) | 55 |
| sgKeap1-#5 | 2 | TTGGCATCATGAACGAGCTGCGG (SEQ ID NO: 5) | 18.8 |
| sgKeap1-#6 | 2 | TGAGGCCAGCACCACCTTGTGGG (SEQ ID NO: 6) | 14.3 |
| sgKeap1-#7 | 2 | CATGGCCTTGAAGACAGGGCTGG (SEQ ID NO: 7) | 43.5 |
| sgKeap1-#8 | 2 | GTGAACATGGCCTTGAAGACAGG (SEQ ID NO: 8) | 79.5 |
| sgKeap1-#9 | 2 | CCATGTTCACCAACGGGCTGCGG (SEQ ID NO: 9) | 1.7 |
| sgKeap 1-#10 | 2 | ATGGAGGTGGTGTCCATTGAGGG (SEQ ID NO: 10) | 81.3 |
| sgKeap1-BR-#1 | 3 | CCAGGTAGCTGAGCGACTGTCGG (SEQ ID NO: 11) | 22.1 |
| sgKeap1-BR-#2 | 3 | GAGGCTTACAACCCCAGTGACGG (SEQ ID NO: 12) | 61.9 |
| sgKeap 1-BR-#3 | 3 | TCATGGGGTTGTAACAGTCCAGG (SEQ ID NO: 13) | 49.7 |
| sgKeap1-BR-#4 | 3 | ACAACCCCATGACCAATCAGTGG (SEQ ID NO: 14) | 59.9 |
| sgKeap1-BR-#5 | 3 | CTGCATCCACCACAACAGTGTGG (SEQ ID NO: 15) | 79 |

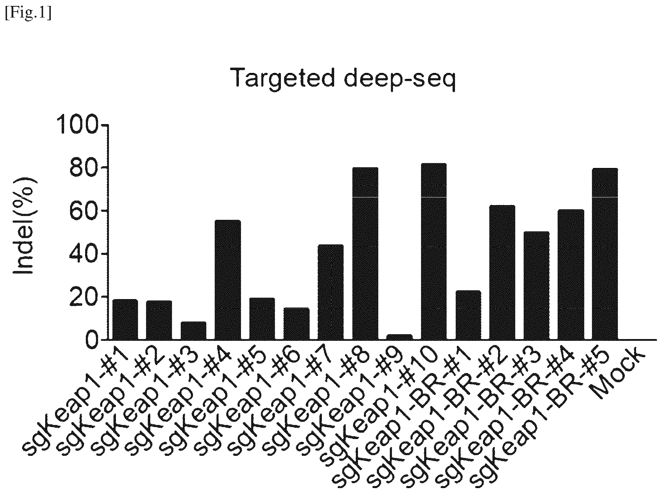

Each guide RNA targeting the target sequences of SEQ ID NOs: 1 to 15 was synthesized and used in subsequent experiments. The indel efficiency of each target sequence for each guide RNA was measured using the targeted deep sequencing method below, and the results are shown in FIG. 1.

Guide RNA was transcribed in vitro using the MEGA short script T7 kit (Ambion) according to the manufacturer's instructions. A template for sgRNA was prepared through annealing and extension of two complementary oligonucleotides.

Ribonucleoprotein (RNP) delivery

A RNP complex were introduced by electroporation using a 4D-Nucleofector (Lonza). Specifically, the RNP complex was formed by mixing 4 ug of Cas9 protein and 4 ug of in vitro transcribed sgRNA (prepared according to the manufacturer's protocol using T7 polymerase (New England BioLabs)), and the mixture was incubated for 10 min at room temperature. The RNP complex was electroporated using the nucleofector program EW-104 together with 20 ul of Primary P1 buffer-treated $4 \times 10^5$ Human bone marrow MSC (Lonza, Cat. No. PT-2501). As a result, mesenchymal stem cells (KEAP1 KO MSC) in which KEAP1 gene was knocked out were obtained.

In this specification, cells in which the SHS231 genetic sequence location was engineered were used as a control for the artificially engineered mesenchymal stem cells. Specifically, a guide RNA capable of targeting the SHS231 target sequence 5'-GATGTGCTCACTGAGTCTGA AGG-3' (SEQ ID NO: 16) (underlined: AM sequence) was synthesized according to the above-described experimental method, and By introducing guide RNA and Cas9 protein in the form of RNP into mesenchymal stem cells, MSC with an engineered SHS231 genetic sequence were obtained.

Targeted deep sequencing

Genomic DNA (gDNA) was extracted from the obtained KEAP1 KO MSC using a blood genomic DNA extraction kit (Favorgen) according to the manufacturer's protocol. To amplify a target region, 100 ng of genomic DNA (gDNA) was amplified using Phusion high-hidelity DNA polymerase PCR Polymerase (NEB). For deep sequencing library generation, amplicons were amplified once more using TruSeq HT dual index primers (Illumina, San Diego, CA, USA). Paired-end sequencing was performed using the Illumina Miniseq System, and the indel frequency was calculated at 'http://www.rgenome.net/'.

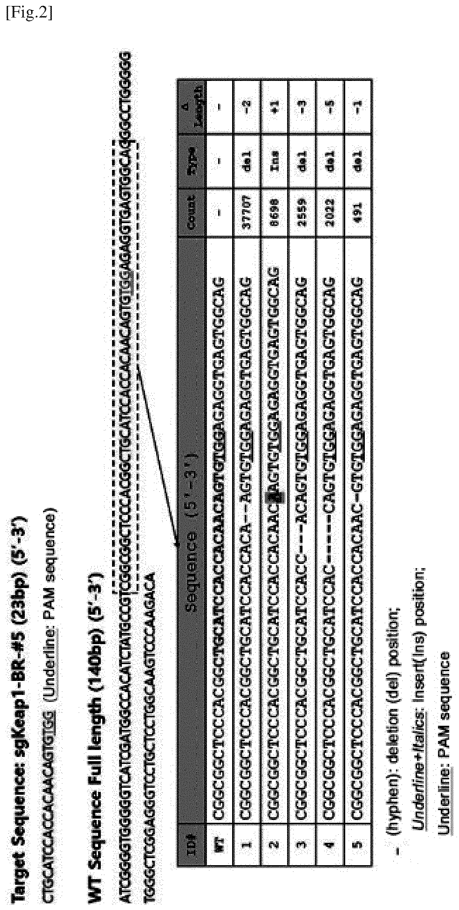

As a result of targeted deep sequencing, the mutation location of KEAP1 KO MSC for each target sequence was indicated based on the sequence of a wild-type KEAP1 gene. As an example, the results of targeted deep sequencing of SEQ ID NO: 15 (sgKeap1-BR-#5) were shown in FIG. 2.

The primer sequences for each target sequence used for targeted deep sequencing are shown in Tables 2 to 4.

[Table 2]

| Classification | Forward primer (5'-3') | Reverse primer (5'-3') |
|---|---|---|
| sgKeap1-#1 | AGCCAGATCCCAGGCCTAGC (SEQ ID NO: 17) | CCGGTGCATCCTGGTACTTG (SEQ ID NO: 18) |
| sgKeap1-#2 | | |
| sgKeap1-#3 | | |
| sgKeap1-#4 | | |
| sgKeap1-#5 | | |
| sgKeap1-#6 | TTGGCATCATGAACGAGCTG (SEQ ID NO: 19) | ACTTCTCGCCCATGGAGATG (SEQ ID NO: 20) |
| sgKeap1-#7 | | |
| sgKeap1-#8 | | |
| sgKeap1-#9 | | |
| sgKeap1-#10 | TGGCCCACAAGGTGGTGCTG (SEQ ID NO: 21) | GGACAACGCTGTCGATCTGG (SEQ ID NO: 22) |
| sgKeap1-BR-#1 | CCTGGTCAAGATCTTCGAGG (SEQ ID NO: 23) | GAGTTGTTCCTGCCGCCCAC (SEQ ID NO: 24) |
| sgKeap1-BR-#2 | | |
| sgKeap1-BR-#3 | GTGGGCGGCAGGAACAACTC (SEQ ID NO: 25) | TCCCTGAAGACAGGAAGAGG (SEQ ID NO: 26) |
| sgKeap1-BR-#4 | | |
| sgKeap1-BR-#5 | | |

[Table 3]

| Classification | Forward primer (5'-3') | Reverse primer (5'-3') |
|---|---|---|
| sgNRF2-#1 | ACATGAGCTCTCTCCTTCCT (SEQ ID NO: 27) | GGGAGAAATTCACCTGTCTCTT (SEQ ID NO: 28) |
| sgNRF2-#2 | | |
| sgNRF2-#3 | | |
| sgNRF2-#4 | | |
| sgNRF2-#5 | CAAGTGATTAGAATGGATGGCTATG (SEQ ID NO: 29) | GGAAGGAGAGAGCTCATGTTT (SEQ ID NO: 30) |
| sgNRF2-#6 | | |
| sgNRF2-#7 | | |
| sgNRF2-#8 | CAACTAGATGAAGAGACAGGTGAA (SEQ ID NO: 31) | GAGGCTGAGGTTGGAAAGTAG (SEQ ID NO: 32) |
| sgNRF2-#9 | | |
| sgNRF2-#10 | | |

[Table 4]

| Classification | Forward primer (5'-3') | Reverse primer (5'-3') |
|---|---|---|
| SHS231-DS | ACACTCTTTCCCTACACGACGCTCT TCCGATCTGACTACAGTAGTTAATC ATC (SEQ ID NO: 33) | GTGACTGGAGTTCAGACGTGTGCTCT TCCGATCTCAGATACAAAAAGGGTTC TC (SEQ ID NO: 34) |

KEAP1 mRNA qRT-PCR Assay

Total RNA samples were obtained using the RNeasy Mini Kit (Qiagen), and cDNA was synthesized from 1 ug of total RNA using the ReverTra Ace qPCR RT Kit (Toyobo). Quantitative RT-PCR was performed on the synthesized cDNA using Power SYBR Green PCR Master Mix (Applied Biosystems) and StepOnePlus Real Time PCR system (Appled biosystems). PCR temperature, time, and cycle number are as follows. DNA denaturation was performed at 95 °C for 10 minutes, primer annealing was performed at 55-60 °C for 30 seconds, polymerization was performed at 72°C for 30 seconds, and amplification was repeated in 40 cycles. Human GAPDH was used as a normalization control. Primer sequences for each gene used in qRT-PCR are shown in Tables 5 and 6.

[Table 5]

| Classification | Forward primer (5'-3') | Reverse primer (5'-3') |
|---|---|---|
| Keap1 | CCTCAATCGTCTCCTTTATGCC (SEQ ID NO: 35) | GATCATTCGCCACTCGTTCC (SEQ ID NO: 36) |

[Table 6]

| Classification | Forward primer (5'-3') | Reverse primer (5'-3') |
|---|---|---|
| NRF2 | CACATCCAGTCAGAAACCAGTGG (SEQ ID NO: 37) | GGAATGTCTGCGCCAAAAGCTG (SEQ ID NO: 38) |

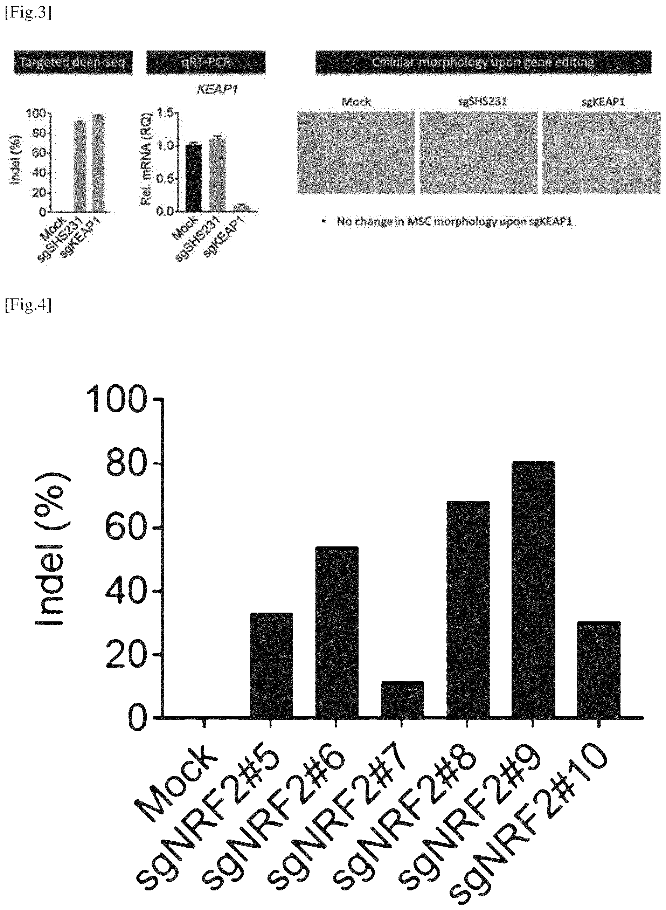

By screening Keap1 target sgRNA, a high level of indel was found at the target sequence (SEQ ID NO: 15) within KEAP1 gene, and the expression of KEAP1 mRNA was also found to be lowered to close to 0. In addition, when comparing WT, control, and Keap1 KO MSCs, there was no significant difference in appearance between spindle-like and fibroblast-like shapes (see FIG. 3).

**Comparative Example 1: Preparation of NRF2-edited mesenchymal stem cells**

Exon 2 of the NFE2L2 gene, which encodes the NRF2 protein, is related to KEAP1 interacting domain, and it is known that when a mutation in which the NFE2L2 exon 2 is deleted occurs, the target gene of Nrf2 is activated (Leonard D Goldstein et. al., Cell Rep. 2016 Sep 6;16(10):2605-2617). A comparative experiment was performed to confirm whether the oxidative stress resistance of KEAP1 KO MSCs was due to KEAP1 KO or increased activity of Nrf2. In order to create Nrf2 active model cells, gene editing was performed to prepare mesenchymal stem cells (Nrf2 edited MSC) in which exon 2 of NRF2 was skipped, and a comparison experiment of $H_2O_2$ (oxidative stress) resistance of KEAP1 KO MSC and Nrf2 edited MSC was performed.

The specific experimental method is as follows.

sgRNA design

To target the NRF2 gene, a guide RNA that corresponds to 1-0-0 mismatches (0, 1, 2) in the off-target profile and may target intron 1 or intron 2 was selected.

The target sequence of the NRF2 gene is summarized in Table 7 below.

[Table 7]

| Classification | RGEN Target (5' to 3' direction), with PAM(underlined) |
|---|---|
| sgNRF2-#1 | ATTTGATTGACATACTTTGGAGG (SEQ ID NO: 39) |
| sgNRF2-#2 | TGGAGGCAAGATATAGATCTTGG (SEQ ID NO: 40) |
| sgNRF2-#3 | TATTTGACTTCAGTCAGCGACGG (SEQ ID NO: 41) |
| sgNRF2-#4 | GCGACGGAAAGAGTATGAGCTGG (SEQ ID NO: 42) |
| sgNRF2-#5 | CACTGTTGATGGTGGGAAGTGGG (SEQ ID NO: 43) |
| sgNRF2-#6 | ATTATGCCACTGTTGATGGTGGG (SEQ ID NO: 44) |
| sgNRF2-#7 | CATTATGCCACTGTTGATGGTGG (SEQ ID NO: 45) |
| sgNRF2-#8 | GTACAGAGTACTCAGTTCTTGGG (SEQ ID NO: 46) |
| sgNRF2-#9 | GTTCTTGGGAAAGTTATGGCAGG (SEQ ID NO: 47) |
| sgNRF2-#10 | GGAAAGTTATGGCAGGTTTAAGG (SEQ ID NO: 48) |

sgNRF2#1-#4 uses NRF2 exon2 (KEAP1 interacting exon) as a target site, and sgNRF2#5-#10 uses an NRF2 intron site (#6 and #9 used simultaneously to induce large deletion of NRF2 exon 2) as a target site.

Among the target sequences, sgRNAs targeting SEQ ID NOs: 43 to 48 were synthesized, and the indel efficiency for the NRF2 gene was measured using the same targeting deep sequencing method as in Example 1, and the results are shown in FIG. 4.

In two types of in-vitro transcribed sgRNAs that may target the target sequences of SEQ ID NO: 44 and SEQ ID NO: 47, respectively, the above two types of sgRNA and editor proteins are simultaneously introduced into one mesenchymal stem cell using the same electroporation method as Example 1 above to prepare MSCs in which exon 2 of the Nrf2 gene was skipped.

**Example 2: Preparation of KEAP1 knocked-out mesenchymal stem cells**

sgRNA design

The target sequence of KEAP1 gene was selected in the same manner as in Example 1 and is shown in Table 8 below.

[Table 8]

| Classification | Exon | RGEN Target (5' to 3' direction) with PAM(underlined) | Indel (%) |
|---|---|---|---|
| sgKeap1_#31 | 2 | GTACGCCTCCACTGAGTGCAAGG (SEQ ID NO: 49) | 20.5 % |
| sgKeap1_#23 | 3 | ACAGCGACGGTTCTACGTCCAGG (SEQ ID NO: 50) | 69.7 % |

(continued)

| Classification | Exon | RGEN Target (5' to 3' direction) with PAM(underlined) | Indel (%) |
|---|---|---|---|
| sgKeap1_#32 | 3 | GCAGTCCGACTCCCGCTGCAAGG (SEQ ID NO: 51) | 26.2 % |
| sgKeap1_#59 | 3 | CTGTCGGAAGTAGCCGCCCGCGG (SEQ ID NO: 52) | 7.2 % |
| sgKeap1_#89 | 3 | CTGTCGGAAGTAGCCGCCCGGGG (SEQ ID NO: 53) | 9.0 % |
| sgKeap1_#108 | 3 | GTTACGGGGCACGCTCATGGGGG (SEQ ID NO: 54) | 41.3 % |
| sgKeap1_#114 | 3 | CGTGCCCCGTAACCGCATCGGGG (SEQ ID NO: 55) | 29.1 % |
| sgKeap1_#117 | 3 | CCCACCCCGATGCGGTTACGGGG (SEQ ID NO: 56) | 14.9 % |

RNP (Ribonucleoprotein) delivery, targeted deep sequencing, and KEAP1 mRNA qRT - PCR Assay

Ribonucleoprotein (RNP) delivery, targeted deep sequencing, and KEAP1 mRNA qRT-PCR Assay were performed in the same manner as Example 1 above.
The primer sequences for each target sequence used for targeted deep sequencing are shown in Table 9.

[Table 9]

| Classification | Forward primer (5'-3') | Reverse primer (5'-3') |
|---|---|---|
| sgKeap1_#31 | AGCCAGATCCCAGGCCTAGC (SEQ ID NO: 17) | CCGGTGCATCCTGGTACTTG (SEQ ID NO: 18) |
| sgKeap1_#23 | CTGAACGTGCGCTGCGAGTC (SEQ ID NO: 57) | AGATCAGGCGGCCCACCTTG (SEQ ID NO: 58) |
| sgKeap1_#32 | | |
| sgKeap1_#59 | CCTGGTCAAGATCTTCGAGG (SEQ ID NO: 23) | GAGTTGTTCCTGCCGCCCAC (SEQ ID NO: 24) |
| sgKeap1_#89 | GACAGTCGCTCAGCTACCTG (SEQ ID NO: 59) | GCGACCACTGATTGGTCATG (SEQ ID NO: 60) |
| sgKeap1_#108 | GTGGGCGGCAGGAACAACTC (SEQ ID NO: 25) | CTCAGTGTCTTGGGACTTGC (SEQ ID NO: 61) |
| sgKeap1_#114 | | |
| sgKeap1_#117 | | |

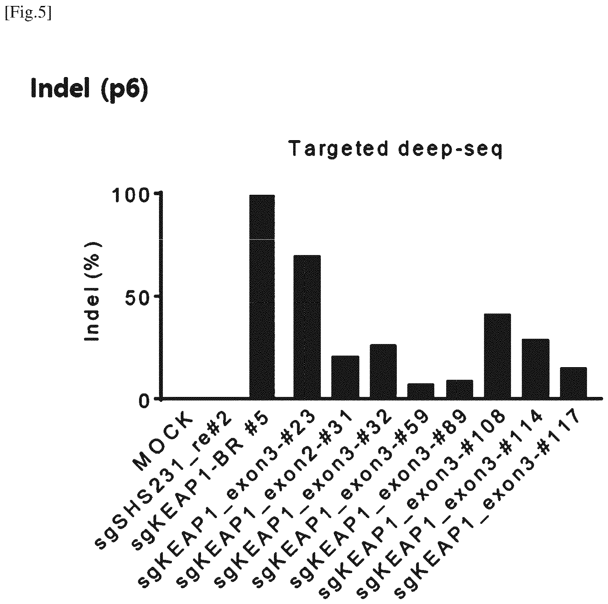

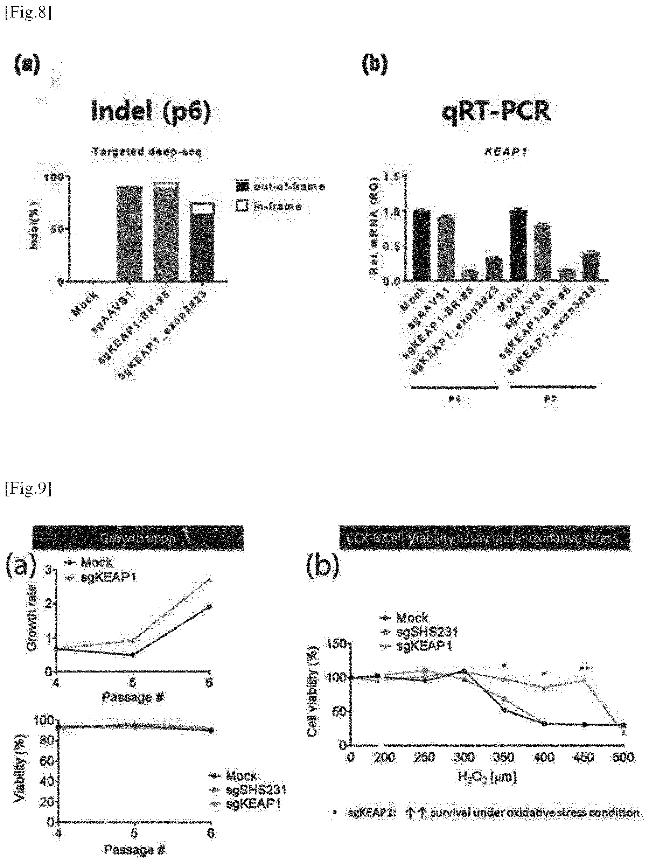

The indel efficiency of each target sequence for each guide RNA targeting the target sequences of SEQ ID NO: 15 and SEQ ID NOs: 49 to 56 was measured using the following targeted deep sequencing method, and the results are shown in FIG. 5 (in FIG. 5, #BR5 is synthetic sgRNA (synthego) and the rest are in vitro transcribed RNA (IVT-RNA)).

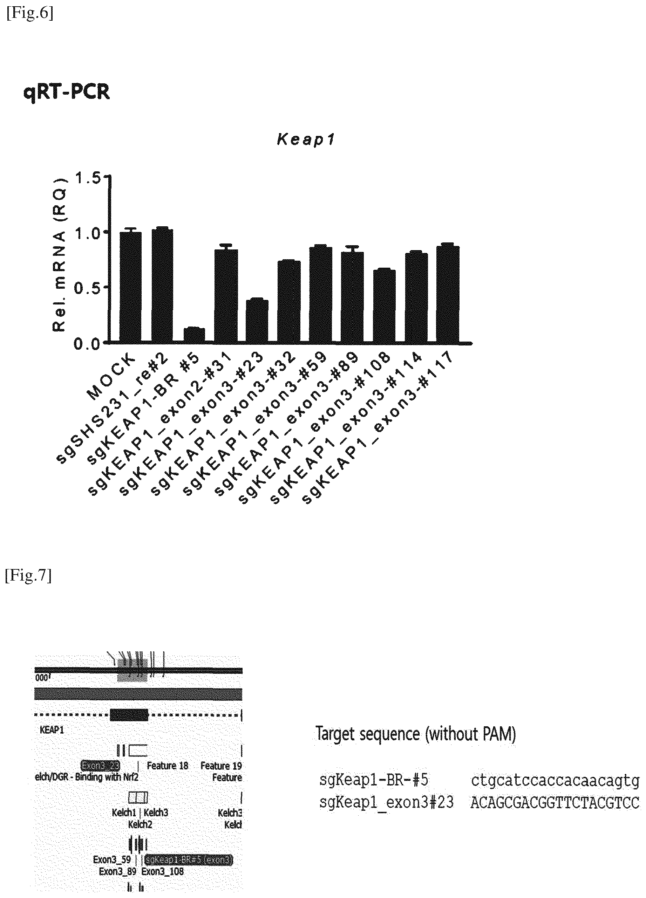

FIG. 6 is a graph showing comparison of the expression level of mRNA transcribed from KEAP1 gene in mesenchymal stem cells in which KEAP1 gene was knocked out using sgRNA targeting each target sequence of KEAP1 gene using qRT-PCR.
Indel efficiency of each target sequence for each guide RNA targeting the target sequences of SEQ ID NO: 15 and SEQ ID NO: 50 in KEAP1-knocked out mesenchymal stem cell □6 (FIG. 8(a)); and the expression level of mRN□ transcribed from □□□1 gene measured using qRT-PCR in KEAP1-knocked out mesenchymal stem cells P6 and P7 (FIG. 8(b)) is shown in FIG. 8.

**Experimental Example 1: Cell viability and growth rate analysis**

CCK8 cell viability assay under oxidative stress

To check the survival rate of cells due to oxidative stress, the surviving cells of each group were measured using Cell Counting Kit-8 (CCK-8). Specifically, cells cultured in a 96-well plate were treated with $H_2O_2$ at different concentrations and cultured for an additional 24 h, then 10 $\mu$L of CCK-8 solution was added to each well and reacted for 2 h. Thereafter, absorbance was measured at 450 nm using an ELISA reader.

Population doubling level (PDL) and Population doubling time (PDT)

To determine the growth rate of cells, the number of cells was measured before and after passaged culture. The value obtained by dividing the harvest cell count (Ct) by the seeding cell count (Ci) was regarded as the growth rate, and PDL (n) was calculated using the following Expression:

[Expression 1]

$$2^n = \frac{C_t}{C_i}$$

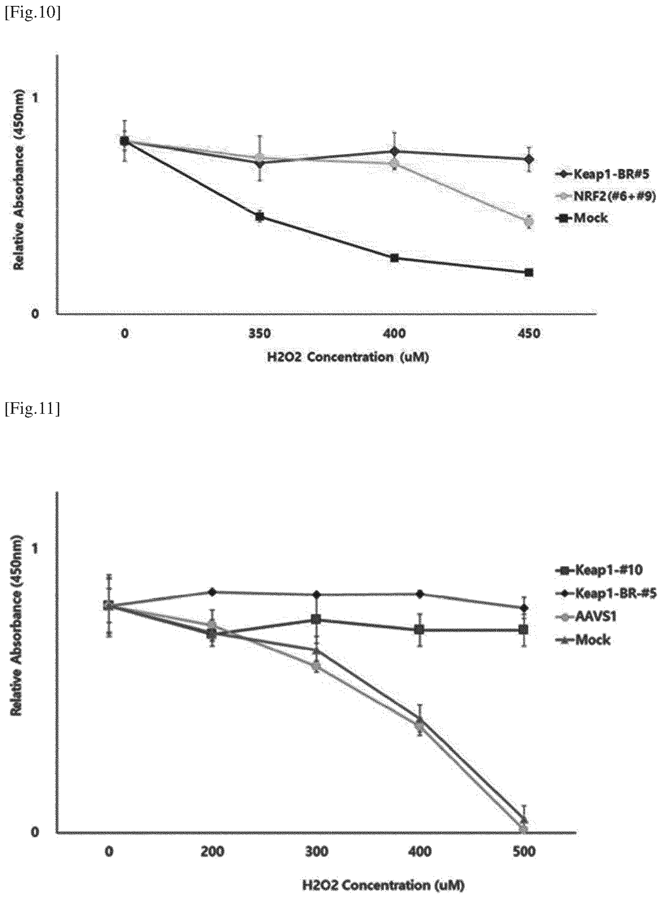

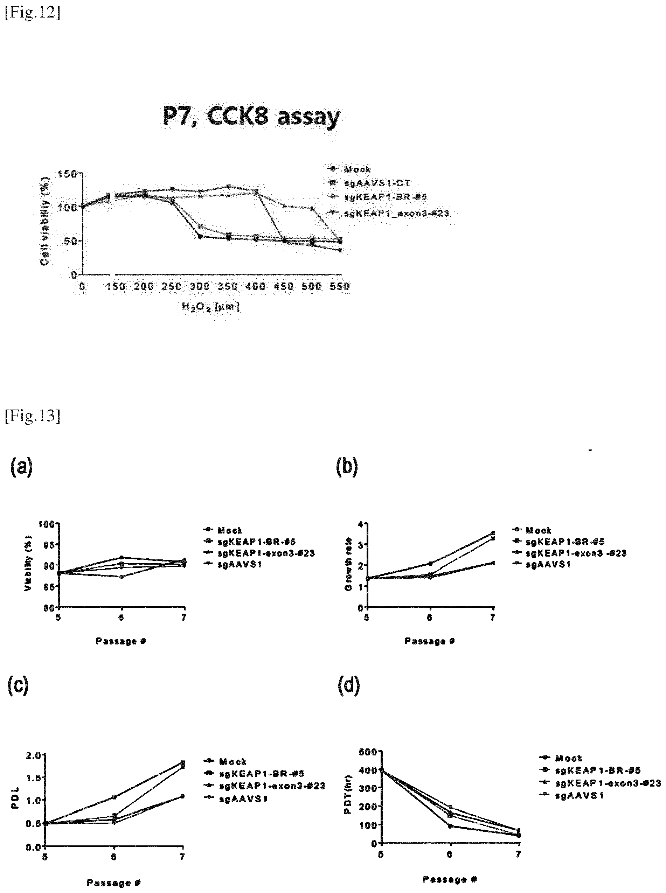

The culture time (hr) divided by PDL was expressed as population doubling time (PDT).
Although the growth rate of KEAP1 KO MSC was observed to be somewhat higher than that of WT, there was no significant difference in viability in the general environment (see FIG. 9(a)). On the other hand, as a result of gradually increasing concentrations of hydrogen peroxide (creating an oxidative stress environment), KEAP1 KO MSCs showed high viability even when a significant amount of hydrogen peroxide was administered (450 $\mu$M) (see FIG. 9(b)).
NRF2 exon2 skip edited MSC (co-targeting sgNRF2-#6+#9) showed a similar survival effect up to 400 uM of $H_2O_2$ as KEAP1 KO MSC (targeting sgKeap1-BR#5), but KEAP1 KO MSC showed a higher survival effect at 450 uM of $H_2O_2$. This shows that KEAP1 knockout has other survival effects on $H_2O_2$ resistance as well as NRF2 activation (see FIG. 10).
As a result of comparing $H_2O_2$ resistance according to KEAP1 target sequence, mesenchymal stem cells knocking out the target sequence of SEQ ID NO: 15 (sgKeap1-BR#5) showed a relatively higher survival effect at high concentrations of $H_2O_2$ (see FIGs. 11 and 12).
Survival rate (FIG. 13(a)), growth rate (FIG. 13(b)), population doubling level (PDL) (FIG. 13(c)), and population doubling time (PDT) (FIG. 13(d)) of stem cells knocking out the target sequences of SEQ ID NO: 15 (sgKeap1-BR#5) and SEQ ID NO: 50 (sgKEAP1-exon3-#23) are shown in FIG. 13.

**Experimental Example 2: Cell cycle analysis**

Bromodeoxyuridine (BrdU) assay

Before harvesting the cultured cells, the cells were treated with 10 uM of BrdU for 1 hour. After treatment with BrdU, the cells were fixed with 3% formaldehyde diluted in PBS for 1 hour at 4 °C, then harvested and treated with 1% Triton X-100 for 5 min at room temperature. The cells were centrifuged again, washed with PBS, treated with 4N HCL at room temperature for 10 min to loosen the DNA double strands, and then washed with PBS. The cells were treated with blocking solution (30% FBS, 1% BSA, 0.01% Tween 20 in PBS) for 30 min at room temperature and then harvested. Anti-BrdU mouse IgG diluted 100 times in PBS was reacted at 4 °C for 30 min. After washing with 0.2% tween 20 diluted in PBS and centrifuging, the collected cells were finally diluted with PBS and analyzed by flow cytometry.

FACS analysis

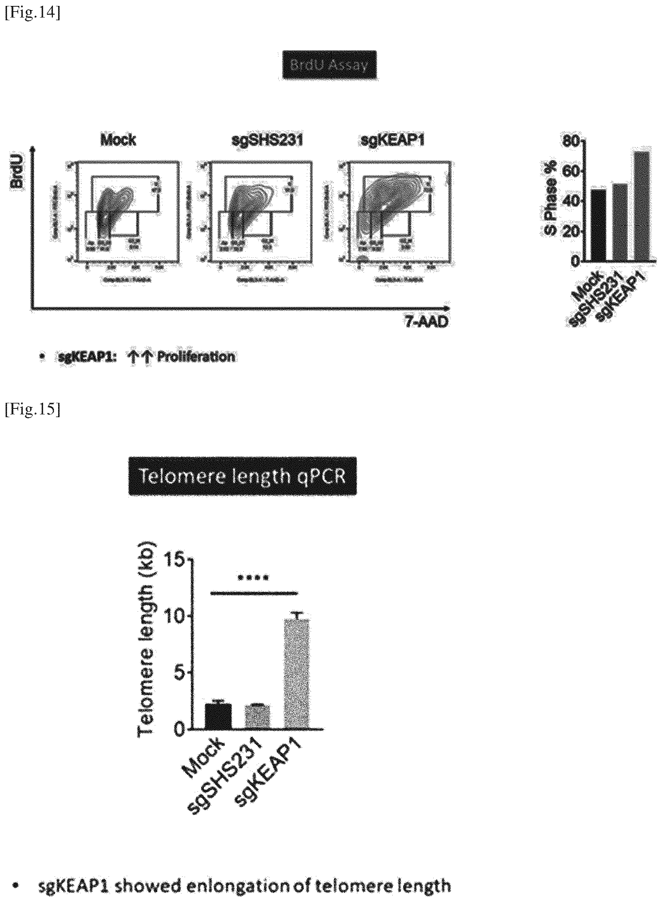

After washing the BrdU-treated KEAP1 KO MSC twice with phosphate buffer saline (PBS), cells were removed from the culture plate using 0.05% trypsin-EDTA and centrifuged at 1,000 rpm for 5 min. Cells were suspended in 100 ul of FACS staining buffer, mixed with antibodies, reacted at 4 °C for 1 hour, washed twice with PBS, and suspended in 500 ul of PBS for FACS analysis.
As a result of observing the cell cycle using the BrdU assay, it was found that S phase was frequently observed in KEAP1 KO MSCs and cell proliferation occurred actively (see FIG. 14).

**Experimental Example 3: Telomere length analysis (Telomere length qPCR)**

Genomic DNA (gDNA) samples of KEAP1 KO MSCs were obtained using the Blood Genomic DNA Extraction Kit (FAVORGEN), and the telomere length value of 5 ng of gDNA was measured using the Absolute Human Telomere Length Quantification qPCR Assay Kit (Sciencell). The lengths of the reference human genomic DNA in the kit and the experimental gDNA sample were compared by performing quantitative RT-PCR using the StepOnePlus Real Time PCR system (Appled biosystems). PCR temperature, time, and cycle number are as follows. DNA denaturation was performed at 95 °C for 10 minutes, primer annealing was performed at 2 °C for 20 seconds, polymerization was performed at 72°C for 45 seconds, and amplification was repeated in 32 cycles.

As a result of telomere length analysis, it was found that as a result of the anti-aging effect of KEAP1 knockout, cell proliferation was active and telomere length was increased (see FIG. 15).

**Experimental Example 4: Proteomics data verification using cytokine analysis**

Cytokine analysis was performed using the Human XL Cytokine Array Kit (Cat. No. ARY022, R & D systems, USA) according to the manufacturer's manual. First, an array separator was placed in a 4-well multi-dish and blocked for 1 h on a rocking platform shaker with array buffer 6. After blocking, the separator was incubated with KEAP1 KO MSC culture fluid sample overnight while maintaining the temperature at 2 to 8 °C on a rocking platform shaker. Thereafter, the separator was washed three times for 10 min each with 1X wash buffer. 1.5 ml of diluted detection antibody cocktail was added to each well and incubated on a shaker for 1 h. Thereafter, the cocktail was washed three times with 1X wash buffer. After washing, 2 ml of streptavidin-HRP was added to each well and reacted for 30 min. Finally, 1 ml of Chemi Reagent Mix solution was sprayed uniformly on each separator and then autoradiometric analysis was performed. The obtained X-ray film was scanned, and the pixel density on the film was analyzed using image analysis software (Image J).

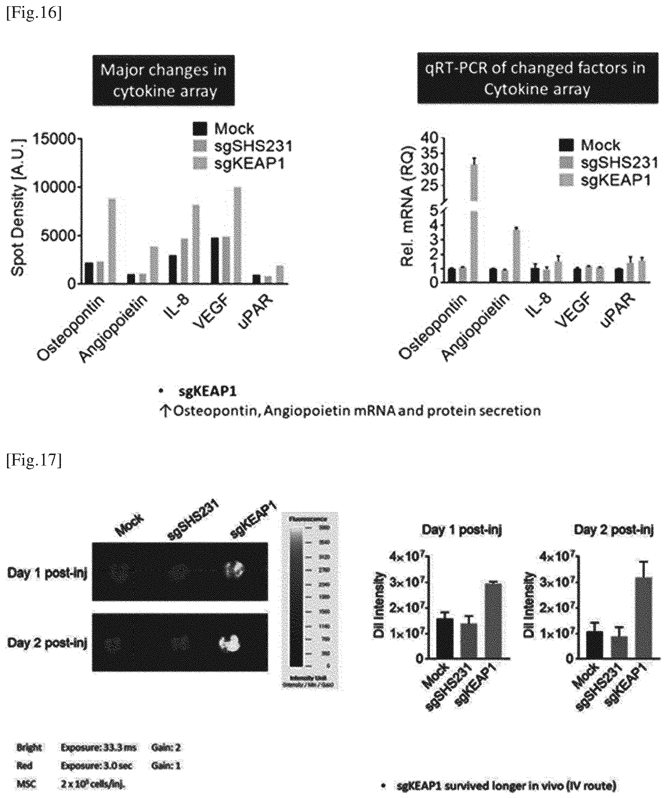

KEAP1 KO MSCs showed increased expression of several cytokines such as Osteopontin (OPN), angiopoietin, IL-8, VEGF, and uPAR, and among them, the expression of ssteopontin and angiopoietin was greatly increased (see FIG. 16). A temporary increase in osteopontin (OPN) promotes angiogenesis, which is beneficial for wound healing, and since angiopoietin-1, whose expression is greatly increased, has a strong vascular protective effect, such as preventing plasma leakage, vascular inflammation, and endothelial cell necrosis, and is expected to have a positive effect on various cardiovascular diseases.

**Experimental Example 5: Confirmation of in vivo viability**

Since MSCs mainly accumulate in the lungs when administered intravenously (IV), KEAP1 knockout MSCs were administered IV to normal mice, and lung tissues were collected, stained, and analyzed. The results are shown in FIG. 11. Specifically, control and KEAP1 KO MSCs were stained with Vybrant DiD cell-labeling solution to track the extent of accumulation in various parts of the body when administered by IV route in vivo (ThermoFisher). The stained MSCs of each experimental group ($2 \times 10^5$ cells) were injected into 6-8 week old C57B1/6 mice (Orient Bio) through the tail vein. On the day 1 and day 2 days after injection, the lungs of the mice were removed, and ex vivo imaging of DiD was performed using the FOBI Fluorescence In vivo imaging system (CELLGENTEK). The residual degree of MSCs was measured by measuring DiD fluorescence intensity.

Referring to FIG. 17, KEAP1 knockout mesenchymal stem cells showed excellent viability in an in vivo environment compared to comparison subjects. It is believed that KEAP1 knockout mesenchymal stem cells, which show such excellent effects, can be used to treat various ischemic and inflammatory diseases in the in vivo environment such as the heart, blood vessels, lungs, and brain.

**Claims**

1. Artificially engineered mesenchymal stems cells comprising an artificially engineered KEAP1 gene, wherein the artificially engineered Keapl gene is different from KEAP 1 gene sequence of a wild-type mesenchymal stem cell, the artificially engineered Keap1 gene includes one or more indels in the nucleic acid sequence, the indel is located within a protospacer-adjacent motif (PAM) sequence in the exon 2 region or exon 3 region of KEAP1 gene, or within a contiguous sequence of 5 to 50 nucleotides adjacent to the 5' or 3' end of the PAM sequence, and the artificially engineered mesenchymal stems cells has improved oxidative stress resistance.

2. The artificially engineered mesenchymal stem cells of claim 1, wherein a sequence of the artificially engineered Keapl gene does not include one or more sequences selected from the group consisting of SEQ ID NOs: 1 to 15 and SEQ ID NOs: 49 to 56.

3. The artificially engineered mesenchymal stem cells of claim 1, wherein, in the artificially engineered mesenchymal stem cell, an mRNA transcribed from the artificially engineered Keap 1 gene has a lower mRNA expression level or a different sequence compared to the mRNA expression level transcribed from the Keap 1 gene of the wild-type mesenchymal stem cell.

4. The artificially engineered mesenchymal stem cells of claim 1, wherein the artificially engineered stem cells have high survival in an oxidative stress environment.

5. The artificially engineered mesenchymal stem cells of claim 1, wherein the artificially engineered mesenchymal stem cells is derived from fat, bone marrow, umbilical cord, placenta, amniotic fluid, amniotic membrane, tissue, umbilical cord blood, or perinatal tissue.

6. A composition for preparing mesenchymal stem cells with oxidative stress resistance, the composition comprising:

   a guide nucleic acid including a guide sequence capable of targeting a target sequence of KEAP1 gene of mesenchymal stem cells, or a nucleic acid sequence encoding the same; and
   an editor protein or a nucleic acid sequence encoding the same.

7. The composition of claim 6, wherein the target sequence is one or more sequences selected from the group consisting of SEQ ID NOs: 1 to 15 and SEQ ID NOs: 49 to 56.

8. The composition of claim 6, wherein the composition includes the editor protein and the guide nucleic acid in the form of ribonucleoprotein (RNP).

9. The composition of claim 6, wherein the composition includes a nucleic acid sequence encoding the editor protein and a nucleic acid sequence encoding the guide nucleic acid in the form of one or more vectors.

10. The composition of claim 9, wherein the vector is selected from the group consisting of plasmid, retrovirus, lentivirus, adenovirus, adeno-associated virus, vaccinia virus, poxvirus, and herpes simplex virus.

11. A method of preparing stem cells with oxidative stress resistance, the method comprising:

   (1) introducing a composition for preparing mesenchymal stem cells with oxidative stress resistance including a guide nucleic acid capable of targeting a target sequence of KEAP1 gene or a nucleic acid sequence encoding the same; and an editor protein or a nucleic acid sequence encoding the same into isolated mesenchymal stem cells; and
   (2) editing KEAP1 gene to reduce or suppress the expression or activity of KEAP1 protein by generating an indel in a target sequence of KEAP1 gene located in the genome of the mesenchymal stem cells.

12. The method of claim 11, wherein the target sequence is one or more sequences selected from the group consisting of SEQ ID NOs: 1 to 15 and SEQ ID NOs: 49 to 56.

13. The method of claim 11, wherein the indel is generated to be located within a protospacer-adjacent motif (PAM) sequence in the exon 2 region or exon 3 region of KEAP1 gene or within a contiguous sequence of 5 to 50 nucleotides adjacent to the 5' or 3' end of the PAM sequence.

14. The method of claim 11, wherein the composition includes a nucleic acid sequence encoding the editor protein and a nucleic acid sequence encoding the guide sequence in the form of one or more vectors.

15. The method of 11, wherein the indel is generated by contacting KEAP1 gene located within the genome of the mesenchymal stem cell with a CRISPR/Cas9 complex including Streptococcus pyogenes-derived Cas9 protein and guide RNA that may target the KEAP1 gene.

16. A pharmaceutical composition for preventing or treating an ischemic disease, comprising the artificially engineered mesenchymal stem cells of any one of claims 1 to 5 as an active ingredient.

17. The pharmaceutical composition of claim 16, wherein the ischemic disease is selected from the group consisting of ischemic heart diseases, peripheral artery disease, critical limb ischemia (CLI), thromboangitis obliteran, diabetic peripheral angiopathy, osteonecrosis, mesenteric ischemia, ischemic colitis, ischemic enteritis, acute kidney injury, ischemia-reperfusion injury, ischemic hepatitis, ischemic pancreatitis, ischemic optic neuropathy, chronic obstructive pulmonary disease, acute respiratory distress syndrome (ARDS), COVID-19 infection, neonatal hypoxic-ischemic encephalopathy, or stroke.

**18.** A method of treating an ischemic disease, comprising administering to a mammal a therapeutically effective amount of artificially engineered mesenchymal stem cells of any one of claims 1 to 5.

**19.** The method of claim 18, wherein the ischemic disease is selected from the group consisting of ischemic heart diseases, peripheral artery disease, critical limb ischemia (CLI), thromboangitis obliteran, diabetic peripheral angiopathy, osteonecrosis, mesenteric ischemia, ischemic colitis, ischemic enteritis, acute kidney injury, ischemia-reperfusion injury, ischemic hepatitis, ischemic pancreatitis, ischemic optic neuropathy, chronic obstructive pulmonary disease, acute respiratory distress syndrome (ARDS), COVID-19 infection, neonatal hypoxic-ischemic encephalopathy, or stroke.

**20.** A use of the artificially engineered mesenchymal stem cells of any one of claims 1 to 5 in the manufacture of a medicament for use in the prevention or treatment of an ischemic disease in a mammal.

**21.** The use of claim 20, wherein the ischemic disease is selected from the group consisting of ischemic heart diseases, peripheral artery disease, critical limb ischemia (CLI), thromboangitis obliteran, diabetic peripheral angiopathy, osteonecrosis, mesenteric ischemia, ischemic colitis, ischemic enteritis, acute kidney injury, ischemia-reperfusion injury, ischemic hepatitis, ischemic pancreatitis, ischemic optic neuropathy, chronic obstructive pulmonary disease, acute respiratory distress syndrome (ARDS), COVID-19 infection, neonatal hypoxic-ischemic encephalopathy, or stroke.

[Fig.1]

Targeted deep-seq

[Fig.2]

EP 4 368 703 A1

**Target Sequence: sgKeap1-BR-#5 (23bp) (5'-3')**

CTGCATCCACCACAACAGTGTGG (Underline: PAM sequence)

**WT Sequence Full length (140bp) (5'-3')**

ATCGGGGTGGGGGTCATCGATGGCCACATCTATGCCGTCGGCGGCTCCCACGGCTGCATCCACCACAACAGTGTGGAGAGGTGAGTGGCAGGGCCTGGGGG
TGGGCTCGGAGGGTCCTGCTCCTGGCAAGTCCCAAGACA

| ID# | Sequence (5'-3') | Count | Type | Δ Length |
|---|---|---|---|---|
| WT | CGGCGGCTCCCACGGCTGCATCCACCACAACAGTGTGGAGAGGTGAGTGGCAG | – | – | – |
| 1 | CGGCGGCTCCCACGGCTGCATCCACCACA--AGTGTGGAGAGGTGAGTGGCAG | 37707 | del | -2 |
| 2 | CGGCGGCTCCCACGGCTGCATCCACCACAAC*A*AGTGTGGAGAGGTGAGTGGCAG | 8698 | Ins | +1 |
| 3 | CGGCGGCTCCCACGGCTGCATCCACC---ACAGTGTGGAGAGGTGAGTGGCAG | 2559 | del | -3 |
| 4 | CGGCGGCTCCCACGGCTGCATCCAC-----CAGTGTGGAGAGGTGAGTGGCAG | 2022 | del | -5 |
| 5 | CGGCGGCTCCCACGGCTGCATCCACCACAAC-GTGTGGAGAGGTGAGTGGCAG | 491 | del | -1 |

– (hyphen): deletion (del) position;

*Underline+Italics*: Insert(Ins) position;

Underline: PAM sequence

[Fig.3]

[Fig.4]

[Fig.5]

# Indel (p6)

[Fig.6]

## qRT-PCR

*Keap1*

[Fig.7]

Target sequence (without PAM)

sgKeap1-BR-#5    ctgcatccaccacaacagtg
sgKeap1_exon3#23   ACAGCGACGGTTCTACGTCC

[Fig.8]

[Fig.9]

[Fig.10]

[Fig.11]

[Fig.12]

[Fig.13]

(a)

(b)

(c)

(d)

[Fig.14]

- sgKEAP1: ↑↑ Proliferation

[Fig.15]

- sgKEAP1 showed enlongation of telomere length

[Fig.16]

• **sgKEAP1**
↑Osteopontin, Angiopoietin mRNA and protein secretion

[Fig.17]

**EP 4 368 703 A1**

<table>
<tr><td colspan="2">INTERNATIONAL SEARCH REPORT</td><td colspan="2">International application No.<br>**PCT/KR2022/009918**</td></tr>
</table>

**A. CLASSIFICATION OF SUBJECT MATTER**

**C12N 5/0775**(2010.01)i; **C12N 15/90**(2006.01)i; **C12N 9/22**(2006.01)i; **C12N 15/113**(2010.01)i; **A61K 35/28**(2006.01)i; **A61P 9/10**(2006.01)i

According to International Patent Classification (IPC) or to both national classification and IPC

**B. FIELDS SEARCHED**

Minimum documentation searched (classification system followed by classification symbols)

C12N 5/0775(2010.01); A01K 67/027(2006.01); A61K 35/17(2014.01); A61K 36/16(2006.01); A61P 11/06(2006.01); A61P 9/10(2006.01); C07C 331/20(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: Keap1, 중간엽 줄기세포(mesenchymal stem cell), 인델(indel), CRISPR, 허혈성 질환(ischemic disease), 치료(treatment)

**C. DOCUMENTS CONSIDERED TO BE RELEVANT**

| Category* | Citation of document, with indication, where appropriate, of the relevant passages | Relevant to claim No. |
|---|---|---|
| X<br>Y | HU, Yiling et al. CRISPR/Cas9-induced loss of Keap1 enhances anti-oxidation in rat adipose-derived mesenchymal stem cells. Frontiers in Neurology. 18 February 2020, vol. 10, Article 1311, pp. 1-7.<br>See abstract; pages 1-4; figure 1; and table 4. | 1-15<br>16-21 |
| Y | US 2016-0120158 A1 (THE JOHNS HOPKINS UNIVERSITY) 05 May 2016 (2016-05-05)<br>See paragraphs [0007], [0073]-[0074] and [0083]; and claim 1. | 16-21 |
| A | GUPTA, Dipti et al. Genetic activation of NRF2 by KEAP1 inhibition induces fetal hemoglobin expression and triggers anti-oxidant stress response in erythroid cells. Blood. 13 November 2019, vol. 134, Supplement_1, Article No. 210, pp. 1-3.<br>See entire document. | 1-21 |
| A | US 2011-0250300 A1 (BISWAL, Shyam et al.) 13 October 2011 (2011-10-13)<br>See entire document. | 1-21 |

☑ Further documents are listed in the continuation of Box C.    ☑ See patent family annex.

| | |
|---|---|
| \*   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **28 October 2022** | **01 November 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

International application No.

**PCT/KR2022/009918**

**C.      DOCUMENTS CONSIDERED TO BE RELEVANT**

| Category* | Citation of document, with indication, where appropriate, of the relevant passages | Relevant to claim No. |
|---|---|---|
| A | WO 2017-161172 A1 (IONIS PHARMACEUTICALS, INC.) 21 September 2017 (2017-09-21) See entire document. | 1-21 |

**INTERNATIONAL SEARCH REPORT**

| International application No. |
| --- |
| **PCT/KR2022/009918** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

  a.   ☑   forming part of the international application as filed.

  b.   ☐   furnished subsequent to the international filing date for the purposes of international search (Rule 13*ter*.1(a)),

    ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

**INTERNATIONAL SEARCH REPORT**
Information on patent family members

International application No.

**PCT/KR2022/009918**

| Patent document cited in search report | | | Publication date (day/month/year) | Patent family member(s) | | | Publication date (day/month/year) |
|---|---|---|---|---|---|---|---|
| US | 2016-0120158 | A1 | 05 May 2016 | US | 2020-0120909 | A1 | 23 April 2020 |
| US | 2011-0250300 | A1 | 13 October 2011 | EP | 1959969 | A2 | 27 August 2008 |
| | | | | US | 2015-0080462 | A1 | 19 March 2015 |
| | | | | WO | 2007-005879 | A2 | 11 January 2007 |
| WO | 2017-161172 | A1 | 21 September 2017 | EP | 3429690 | A1 | 23 January 2019 |
| | | | | US | 10961271 | B2 | 30 March 2021 |
| | | | | US | 2019-0077825 | A1 | 14 March 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190069238
- WO 2012093833 A
- US 20130217131 A
- US 7888121 B
- US 8409861 B
- US 6479626 B
- US 6903185 B

- US 7153949 B
- KR 101656236
- KR 101656237
- KR 101706085
- KR 102052286
- KR 102182847

**Non-patent literature cited in the description**

- **XIAOZHEN DAI et al.** Redox and Metabolic Regulator of Stem Cell State and Function. *Trends Mol Med.,* February 2020, vol. 26 (2), 185-200
- **D S YOON et al.** Cellular localization of NRF2 determines the self-renewal and osteogenic differentiation potential of human MSCs via the P53-SIRT1 axis. *Cell Death & Disease,* 2016, vol. 7, 2093
- **YILING HU et al.** CRISPR/Cas9-Induced Loss of Keap1 Enhances Anti-oxidation in Rat Adipose-Derived Mesenchymal Stem Cells. *Front. Neurol,* 18 February 2020
- **MOHAMMAD MOHAMMADZADEH et al.** overexpression in mesenchymal stem cells reduces oxidative stress-induced apoptosis and cytotoxicity. *Cell Stress and Chaperones,* 2012, vol. 17, 553-565

- **SHOUQIN ZHANG et al.** transfection enhances the efficacy of human amniotic mesenchymal stem cells to repair lung injury induced by lipopolysaccharide. *J Cell Biochem.,* February 2018, vol. 119 (2), 1627-1636
- **ZFN, BEERLI et al.** *Nature Biotechnol.,* 2002, vol. 20, 135-141
- □ABO et al. *Rev. Biochem.,* 2001, vol. 70, 313-340
- **ISALAN et al.** *Nature Biotechnol.,* 2001, vol. 19, 656-660
- **SEGAL et al.** *Curr. Opin. Biotechnol.,* 2001, vol. 12, 632-637
- **CHOO et al.** *Curr. Opin. Struct. Biol.,* 2000, vol. 10, 411-416
- Remington's Pharmaceutical Sciences. Mack Publishing Company, 1995
- **LEONARD D GOLDSTEIN.** *Cell Rep.,* 06 September 2016, vol. 16 (10), 2605-2617